# EUROPEAN PATENT SPECIFICATION

(11) **EP 1 474 070 B1**
(45) Date of publication and mention of the grant of the patent: **19.09.2007**
(21) Application number: 03701283.8
(22) Date of filing: 10.01.2003
(51) Int. Cl.: A61F 13/15

(54) **ABSORBENT LAMINATE**
SAUGFÄHIGES LAMINAT
STRATIFIE ABSORBANT

(30) Priority: 17.01.2002 US 50045
(43) Date of publication of application: 10.11.2004
(73) Proprietor: Paragon Trade Brands, Inc., King of Prussia, Pennsylvania 19406 (US)
(72) Inventor: HANSEN, Ebba, A., Cumming, Ga 300041-5659 (US)
(74) Representative: Leonhard, Frank Reimund
(86) International application number: PCT/US2003/000612
(87) International publication number: WO 2003/061524

(56) References cited:
- WO-A-01/34082
- WO-A-99/27879
- WO-A-03/047486
- US-A- 5 425 725
- US-A- 6 068 620
- US-A1- 2003 135 177

## Description

### FIELD OF THE INVENTION

The present invention relates generally to an absorbent core for a disposable absorbent garment, and more particularly to an absorbent core comprising one or more laminates in which one of the layers of the laminate or laminates contains more than about 50% by weight superabsorbent polymer (SAP),and at least another layer of the laminate is a wicking layer, storage layer, acquisition layer, wicking/ distribution layer, or combinations of these layers, or fragmented layers thereof.

### BACKGROUND OF THE INVENTION

Traditionally, disposable absorbent garments such as infant diapers or training pants, adult incontinence products and other such products were constructed with a moisture-impervious outer backing sheet, a moisture-pervious body-contacting inner liner sheet, and a moisture-absorbent core sandwiched between the liner and backing sheets. Much effort has been expended to find cost-effective materials for absorbent cores that display favorable liquid absorbency and retention. Superabsorbent materials in the form of granules, beads, fibers, bits of film, globules, etc., have been favored for such purposes. Such superabsorbent materials generally are polymeric gelling materials that are capable of absorbing and retaining even under moderate pressure large quantities of liquid, such as water and body wastes, relative to their own weight.

The superabsorbent material generally is a water-insoluble but water-swellable polymeric substance capable of absorbing water in an amount which is at least ten times the weight of the substance in its dry form. In one type of superabsorbent material, the particles or fibers may be described chemically as having a back bone of natural or synthetic polymers with hydrophilic groups or polymers containing hydrophilic groups being chemically bonded to the back bone or in intimate admixture therewith. Included in this class of materials are such modified polymers as sodium neutralized cross-linked polyacrylates and polysaccharides including, for example, cellulose and starch and regenerated cellulose which are modified to be carboxylated, phosphonoalkylated, sulphoxylated or phosphorylated, causing the SAP to be highly hydrophilic. Such modified polymers may also be cross-linked to reduce their water-solubility.

The ability of a superabsorbent material to absorb liquid typically is dependent upon the form, position, and/or manner in which particles of the superabsorbent are incorporated into the absorbent core. Whenever a particle of the superabsorbent material and absorbent core is wetted, it swells and forms a gel. Gel formation can block liquid transmission into the interior of the absorbent core, a phenomenon called "gel blocking." Gel blocking prevents liquid from rapidly diffusing or wicking past the "blocking" particles of superabsorbent (e.g., those particles that have swelled and touched an adjacent swelled particle), causing portions of a partially hydrated core to become inaccessible to multiple doses of urine. Further absorption of liquid by the absorbent core must then take place via a diffusion process. This is typically much slower than the rate at which liquid is applied to the core. Gel blocking often leads to leakage from the absorbent article well before all of the absorbent material in the core is fully saturated.

Despite the incidence of gel blocking, superabsorbent materials are commonly incorporated into absorbent cores because they absorb and retain large quantities of liquid, even under load. However, in order for superabsorbent materials to function, the liquid being absorbed in the absorbent structure must be transported to unsaturated superabsorbent material. In other words, the superabsorbent material must be placed in a position to be contacted by liquid. Furthermore, as the superabsorbent material absorbs the liquid it must be allowed to swell. If the superabsorbent material is prevented from swelling, it will cease absorbing liquids.

Adequate absorbency of liquid by the absorbent core at the point of initial liquid contact and rapid distribution of liquid away from this point is necessary to ensure that the absorbent core has sufficient capacity to absorb subsequently deposited liquids. Previously known absorbent cores have thus attempted to absorb quickly and distribute large quantities of liquids throughout the absorbent core while minimizing gel blocking during absorption of multiple doses of liquid.

In general, some of the most important performance attributes of an absorbent core of a diaper (or any other absorbent garment) are functional capacity, rate of absorption, and core stability in use. Absorption under load or AUL is a good measure of functional capacity and the rate at which that absorption occurs. AUL is believed to be a function of both SAP basis weight (mass per unit area) and the composition of SAP used in the composite. Baby diaper cores that contain only fluff pulp and a high gel strength SAP maintain adequate SAP efficiency if the core contains less than about 50% SAP. Fluff/SAP diaper cores containing more than 50% SAP can result in lower SAP efficiency because of gel blocking. Although fluff/SAP cores at greater than 50% SAP can provide adequate absorbency, the overall basis weight of the core must be increased to compensate for the lower efficiency of the SAP. Increasing the basis weight decreases the performance/cost ratio of the absorbent core, making them uneconomical. Also, increased basis weights tend to affect the fit and comfort of the garment, as well as impacting the packaging and shipping costs.

It is known to provide absorbent laminates comprised of, for example, an upper layer, a lower layer, and a central fibrous layer containing from 50% to 95% by weight SAP, as is described in U.S. Patent No. 6,068,620, which also discloses that the upper and lower layers are comprised of tissue, airlaid fluff pulp or synthetic non-woven fibrous layers. The upper and lower layers are said to assist in maintaining the integrity of the core, the laminate layered arrangement is said to minimize gel blocking, and the laminate can be folded in various configurations. It also is known to provide a composite absorbent structure having a wicking layer bonded to the absorbent layer with a bonding agent such that the absorbent structure has a Contact Intimacy Ratio. U.S. Patent No. 6,329,565, the disclosure of which is incorporated by reference herein in its entirety, discloses an absorbent composite having a wicking layer that has a vertical wicking flux value, an absorbent liquid retention layer, and a bonding agent.

It also is known to provide absorbent cores comprised of differing materials in an attempt to maximize comfort and efficiency of the core, and to provide areas having varying degrees of absorbency. U.S. Patent No. 5,849,002, the disclosure of which is incorporated by reference herein in its entirety, discloses absorbent cores having three zones: (i) one zone for receiving fluids; (ii) one zone for distributing and storing fluids; and (iii) one zone for preventing leakage. U.S. Patent No. 5,853,402, the disclosure of which is incorporated by reference herein in its entirety, discloses composite absorbent cores comprising at least an absorbent material and a porous resilient material. Other composite, zoned, or multi-component cores are disclosed in, for example, U.S. Patent Nos. 5,681,300 (blended absorbent core), 5,882,464 (crimping to join two absorbent structures), 5,891,120 (varying SAP concentration throughout core), 5,425,725 and 5,938,650 (multiple fiber free SAP pockets in core), and 5,922,165 (method of joining outer layers with absorbent core disposed between the outer layers). The respective disclosures of each of these documents are incorporated by reference herein in their entirety.

WO 01/34082 discloses a thin absorbent core made from a folded absorbent laminate. The core consists of an upper layer and a lower layer and therebetween an absorbent layer containing, for example, 50% to 95% by weight SAP.

The description herein of advantages and disadvantages of various features, embodiments, methods, and apparatus disclosed in other publications is in no way intended to limit the present invention. Indeed, certain features of the invention may be capable of overcoming certain disadvantages, while still retaining some or all of the features, embodiments, methods, and apparatus disclosed therein.

### SUMMARY OF THE INVENTION

It would be desirable to provide an absorbent garment having an improved ability to retain fluids and consequently, to prevent leakage. It also would be desirable to provide an absorbent core that includes an increased amount of superabsorbent polymers, but at the same does not suffer from gel blocking to an appreciable extent.

It also would be desirable to provide an absorbent core that has the above mentioned characteristics, and in addition has improved acquisition of fluids, and improved distribution and storage of fluids that insult the core.

It is therefore a feature or object of an embodiment of the invention to provide an absorbent garment having an improved ability to retain fluids. It is an additional feature or object of an embodiment of the invention to provide an absorbent garment that includes an absorbent core having SAP particles as a substantial percentage of its basis weight, but at the same time reducing gel blocking, i.e., retaining high SAP efficiency. It is yet a further feature or object of an embodiment of the invention to provide an absorbent garment that includes an absorbent core having high dry and wet strength for processing and in-use performance. An additional feature or object of various embodiments of the invention is providing an absorbent core that includes SAP particles as a substantial percentage of its basis weight, whereby the core has good fluid acquisition, distribution, and storage characteristics.

These and other features or objects of the invention can be achieved by an absorbent article according to claim 1, including a top sheet, a back sheet and an absorbent laminate core disposed between the top sheet and the back sheet. The absorbent laminate core of the invention is comprised of at least four layers, whereby two of the layers are outer layers, (an upper layer and a lower layer) and one of the inner layers is a central fibrous layer containing tow fibers and from 30 to 95% by weight SAP. The absorbent laminate core further includes as the at least one other inner or central layer, a layer selected from a fluid acquisition layer, a distribution layer, a wicking layer, or combinations and fragments of these layers. The at least two inner layers, the central fibrous layer containing tow fibers and SAP, and the at least one other inner layer, are disposed between the upper layer and the lower layer.

In accordance with an additional feature of an embodiment of the invention, there is provided a method of making an absorbent article according to claim 20 that includes preparing a top sheet material and a back sheet material. The method also includes preparing an absorbent laminate core, and disposing the absorbent laminate core between the top sheet and the back sheet. Preparing the absorbent laminate core includes preparing: (i) a central fibrous layer containing tow fibers and from 30 to 95% by weight SAP; and (ii) at least one other layer selected from a fluid acquisition layer, a distribution layer, a wicking layer, or combinations and fragments of each and every one of these layers, between an upper layer and a lower layer.

In addition to the foregoing advantages, the absorbent garment having an absorbent core comprising one or more highly concentrated SAP laminates improves the comfort and fit of the garment. Further, due to the thinness of the resulting product, less packaging material is needed for the same amount of product, and shipping and handling costs are lowered.

These and other features and advantages of the preferred embodiments will become more readily apparent when the detailed description of the preferred embodiments is read in conjunction with the attached drawings.

### BRIEF DESCRIPTION OF THE DRAWINGS

Figure 1 is a partially cut-away view of an embodiment of the present invention, shown with elastic members fully stretched in the main portion of the garment;
Figure 2 is a cross-sectional view of the absorbent garment in Figure 1 taken along line A-A, illustrating one embodiment for the absorbent laminate core of the invention;
Figure 3 is a cross-sectional view of an embodiment for the absorbent laminate core of the invention;
Figure 4 is a cross-sectional view of an embodiment for the absorbent laminate core of the invention;
Figure 5 is a cross-sectional view of an embodiment for the absorbent laminate core of the invention;
Figure 6 is a cross-sectional view of an embodiment for the absorbent laminate core of the invention;
Figure 7 is a cross-sectional view of an embodiment for the absorbent laminate core of the invention;
Figure 8 is a cross-sectional view of an embodiment for the absorbent laminate core of the invention;
Figure 9 is a cross-sectional view of an embodiment for the absorbent laminate core of the invention;
Figure 10 is a cross-sectional view of an embodiment for the absorbent laminate core of the invention;
Figure 11 is a cross-sectional view of an embodiment for the absorbent laminate core of the invention;
Figure 12 is a cross-sectional view of an embodiment for the absorbent laminate core of the invention;
Figure 13 is a cross-sectional view of an embodiment for the absorbent laminate core of the invention;
Figure 14 is a cross-sectional view of an embodiment for the absorbent laminate core of the invention;
Figure 15 is a cross-sectional view of an embodiment for the absorbent laminate core of the invention;
Figure 16 illustrates various preferred configurations by which the absorbent laminate core of the invention may be folded;

### DETAILED DESCRIPTION OF THE PREFERRED EMBODIMENTS

As used herein, the terms "absorbent garment," "absorbent article" or simply "article" or "garment" refer to devices that absorb and contain body fluids and other body exudates. More specifically, these terms refer to garments that are placed against or in proximity to the body of a wearer to absorb and contain the various exudates discharged from the body. A non-exhaustive list of examples of absorbent garments includes diapers, diaper covers, disposable diapers, training pants, feminine hygiene products and adult incontinence products. Such garments may be intended to be discarded or partially discarded after a single use ("disposable" garments). Such garments may comprise essentially a single inseparable structure ("unitary" garments), or they may comprise replaceable inserts or other interchangeable parts.

The present invention may be used with all of the foregoing classes of absorbent garments, without limitation, whether disposable or otherwise. The embodiments described herein provide, as an exemplary structure, a diaper for an infant, however this is not intended to limit the claimed invention. The invention will be understood to encompass, without limitation, all classes and types of absorbent garments, including those described herein. Preferably, the absorbent core is thin in order to improve the comfort and appearance of a garment.

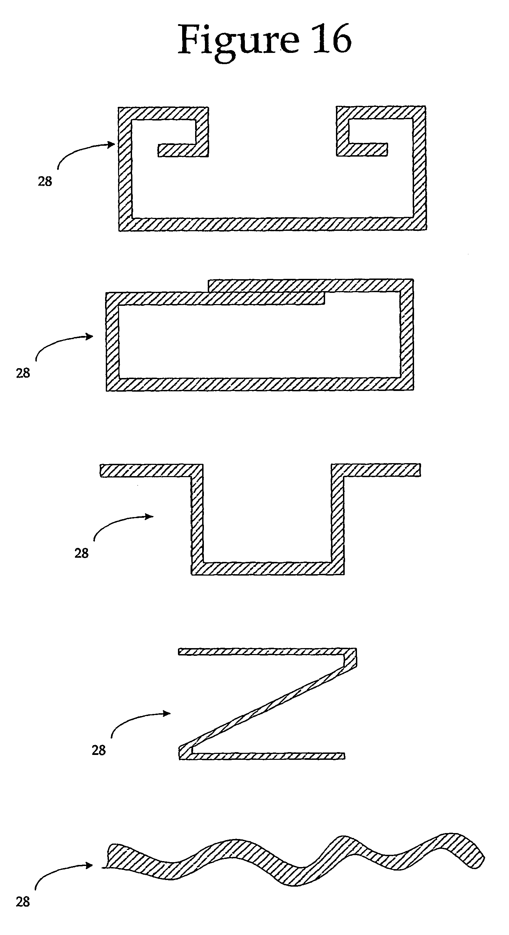

Throughout this description, the expressions *"*upper layer*"* and "lower layer*"* that refer to the layers surrounding the absorbent laminate core of the invention are used merely to describe one layer above the core, and one layer below the core. The upper layer need not always remain vertically above the core, and the lower layer need not always remain vertically below the core. Indeed, many embodiments of the invention encompass various configurations of the absorbent laminate core whereby the laminate is folded in such a manner that the upper layer ultimately becomes the vertically highest and vertically lowest layer at the same time (see, Figure 16). Other configurations are contemplated within the context of the present invention.

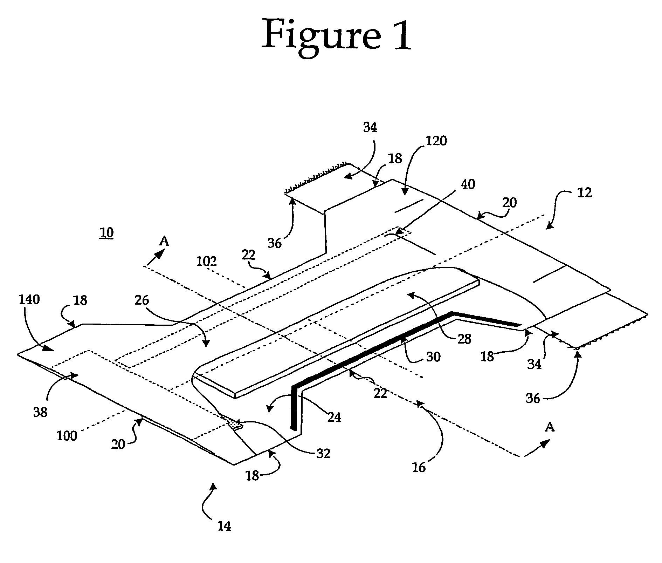

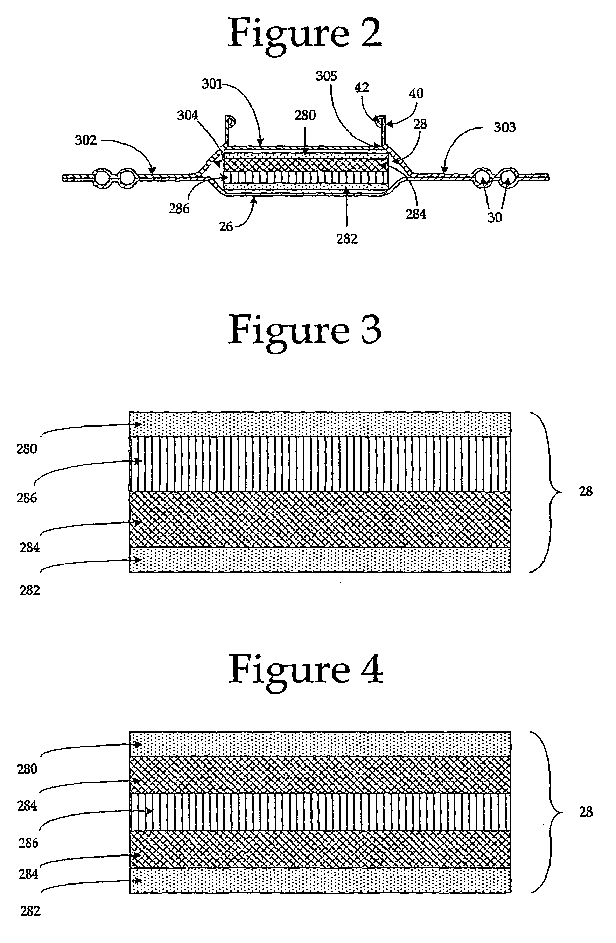

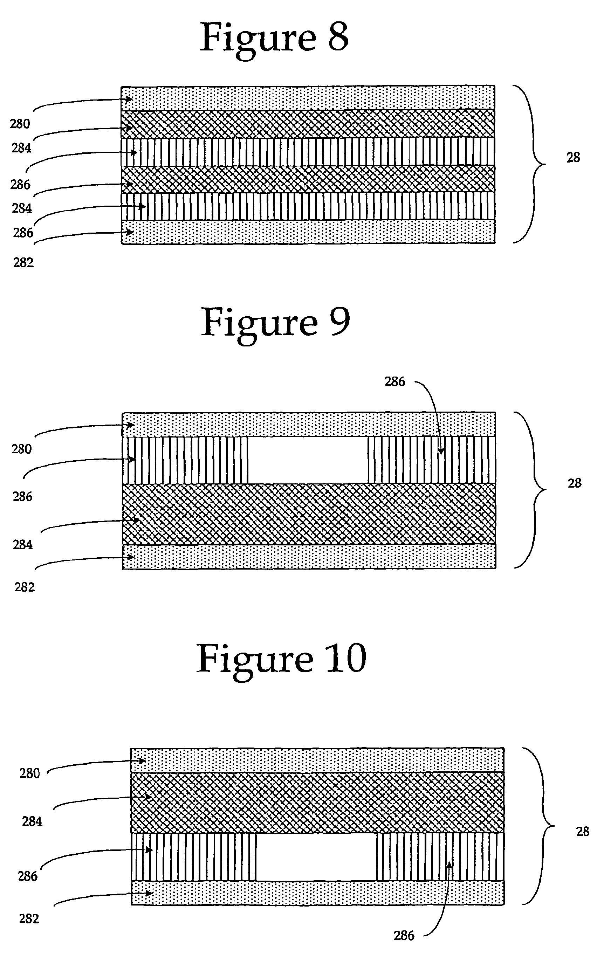

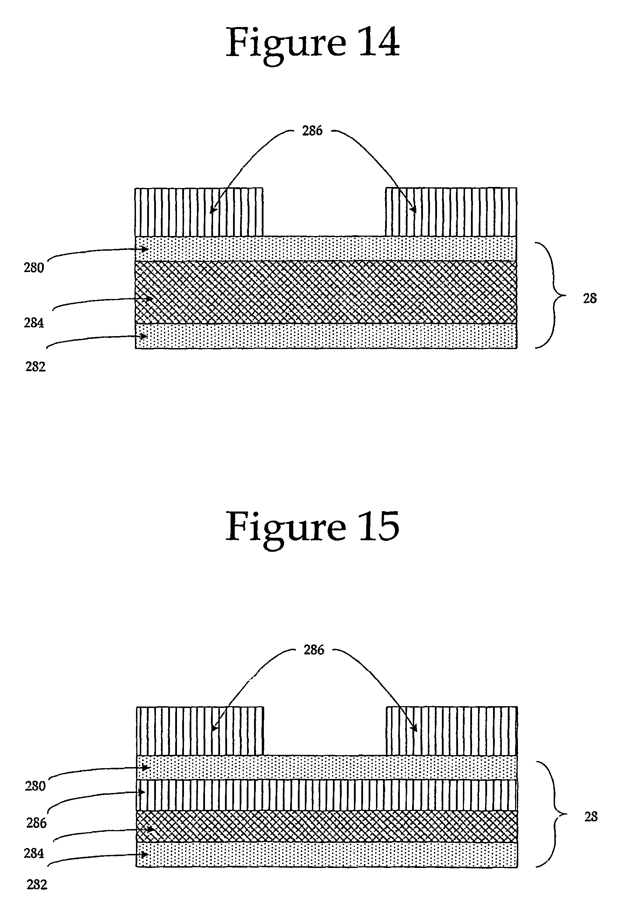

Throughout this description, the expressions "fragmented layer" or "non-continuous layer" denote a layer that does not exist as a unitary article across the relevant cross-section of the absorbent article. For example, absorbent laminate core 28, as shown in Figures 1 and 2, is a unitary article along the longitudinal 100 and lateral 102 axis, throughout its relevant cross-section. A fragmented layer would only exist in certain portions either laterally (as shown in Figures 9, 10, 11, 14, and 15) or longitudinally (not shown). These types of layers enable imparting specific properties to specific areas of the absorbent garment where those properties are needed most.

The term "component" can refer, but is not limited to designated selected regions, such as edges, corners, sides or the like; structural members, such as elastic strips, absorbent pads, stretchable layers or panels, layers of material, or the like; or a graphic.

Throughout this description, the term "disposed" and the expressions "disposed on," "disposing on," "disposed in," "disposed between" and variations thereof (e.g., a description of the article being "disposed" is interposed between the words "disposed" and "on") are intended to mean that one element can be integral with another element, or that one element can be a separate structure bonded to or placed with or placed near another element. Thus, a component that is "disposed on" an element of the absorbent garment can be formed or applied directly or indirectly to a surface of the element, formed or applied between layers of a multiple layer element, formed or applied to a substrate that is placed with or near the element, formed or applied within a layer of the element or another substrate, or other variations or combinations thereof.

Throughout this description, the terms "top sheet" and "back sheet" denote the relationship of these materials or layers with respect to the absorbent composite core. It is understood that additional layers may be present between the absorbent composite core and the top sheet and back sheet, and that additional layers and other materials may be present on the side opposite the absorbent composite core from either the top sheet or the back sheet.

Throughout this description, the expression "tow fibers" relates in general to any continuous fiber. Tow fibers typically are used in the manufacture of staple fibers, and preferably are comprised of synthetic thermoplastic polymers. Usually, numerous filaments are produced by melt extrusion of the molten polymer through a multi-orifice spinneret during manufacture of staple fibers from synthetic thermoplastic polymers in order that reasonably high productivity may be achieved. The groups of filaments from a plurality of spinnerets typically are combined into a tow which is then subjected to a drawing operation to impart the desired physical properties to the filaments comprising the tow. It is believed that tow adds surface area to the core, which improves capacity and capillarity as well as surfaces for glue to attach SAP. Tow also is believed to add wet integrity to the core that would otherwise be very poor, as well as add dry integrity that helps with the manufacturing processes.

The present invention relates generally to absorbent articles, and in particular to an absorbent article that contains a top sheet, a back sheet, and an absorbent laminate core disposed between the top sheet and the back sheet. The absorbent laminate core of the invention preferably is comprised of at least four layers, whereby two of the layers are outer layers, and one of the inner layers preferably is a central fibrous layer containing from about 30 % by weight to about 95% by weight SAP, and tow fibers. The absorbent laminate core further includes as the at least one other inner or central layer, a layer selected from an acquisition layer, a distribution layer, an additional fibrous layer optionally containing SAP, a wicking layer, a storage layer, or combinations and fragments of these layers. The at least two inner layers, the central fibrous layer containing a mixture of tow fibers and SAP, and the at least one other inner layer, are disposed between an upper layer and a lower layer.

The absorbent article of the invention preferably has a front waist region, a rear waist region and a crotch region positioned between the front and rear waist regions. The front waist region and rear waist region can be associated with one another to form a waist opening, and two leg openings. Those skilled in the art recognize that "front" and "rear" in the context of the invention denote for clarity purposes only the front and rear of a user, and that the absorbent article could be reversed whereby the previously described "front" portion becomes the rear portion, and vice versa.

Leg elastics preferably are provided along the leg openings for securely holding the leg openings against the thighs of the wearer to improve containment and fit. A fastening system, either re-sealable or permanent, preferably holds the absorbent article around the wearer's waist. The fastening system assists in associating the front waist region with the rear waist region. A pair of stand-up leg gathers or waist containment flaps may be attached to or formed from the body's side surface of the top sheet.

The absorbent article of the invention includes an absorbent laminate core comprising at least four layers. Each absorbent core laminate preferably comprises at least four layers, a high efficiency, SAP-containing central fibrous layer disposed between upper and lower tissue layers. The absorbent core laminate also includes at least one more layer, which is at least one layer selected from a fluid acquisition layer, a distribution layer, a wicking layer, or combinations and fragments of these layers.

Other non-SAP-containing roll good materials such as latex or thermally bonded airlaid fluff pulp, (e.g., roll good available from Walkisoft, Merfin or Fort James), or synthetic spunbonded, carded, or hydro-entangled non-woven may be positioned above and below the absorbent core. At least the central fibrous layer of the absorbent laminate core preferably contains 50-95% by weight particulate or fibrous SAP and at least one other fibrous or particulate material that is capable of maintaining high SAP efficiency. As described in U.S. Patent No. 6,068,620, SAP efficiency can be expressed as the ratio of the actual SAP absorbency under load, or AUL (expressed as grams of saline absorbed per gram of SAP in the laminate), and the maximum SAP AUL obtained under ideal conditions of low basis weight where gel blocking does not occur. SAP concentrations of from about 30 to about 95% by weight provide thinner roll good composites for efficient shaping and handling. High SAP concentrations also provide thinner absorbent cores that can provide new options for product design. The absorbent laminate cores of the invention can be made using either a wet or dry process.

Forming the absorbent laminate core of the invention with one or more inner layers disposed between an upper and lower layer is believed to decouple key performance attributes of traditional absorbent cores. As recognized by skilled artisans, the various layers of an absorbent core typically are designed with competing interests. A compromise usually is made at the sacrifice of the optimal performance attributes of each of the individual layers. By decoupling the performance attributes of the individual layers, the absorbent laminate core of the preferred embodiments optimizes the key characteristic performance attributes of each of the laminated inner layers, thereby resulting in overall improved performance over previously known absorbent cores, or absorbent laminates.

Stated more specifically, outer layers of absorbent cores generally are designed for optimal wet/ dry strength, liquid acquisition and distribution, as well as SAP containment. The inner layers of absorbent cores generally are designed for optimal absorbency and SAP efficiency. Designers of absorbent cores in the past have had to combine the attributes of the outer and inner layers into a homogeneous composite, often leading to an unacceptable compromise. The absorbent laminate core of the embodiments of the present invention, however, has more than one inner layer, whereby at least one of the inner layers is a high density SAP layer, and this configuration is believed to allow the absorbent laminate core to be designed without compromising performance attributes of the individual components. For example, significant SAP efficiency improvement is realized when one or more laminated layers is employed.

The invention now will be described with reference to the attached drawings illustrating preferred embodiments of the invention. For clarity, features that appear in more than one Figure have the same reference number in each Figure.

Figure 1 is a partially cut away depiction of an exemplary embodiment of an absorbent garment 10 (preferably a disposable absorbent garment) of the present invention. The embodiment shown in Figure 1 is an infant's diaper, however, this depiction is not intended to limit the invention, and those skilled in the art appreciate that the invention covers other types of absorbent articles. For simplicity, however, the invention will be described with reference to an infant's diaper. The garment 10 of Figure 1 is depicted in a generally flattened position, with the body-facing side facing down, and with the various elastic components depicted in their relaxed condition with the effects of the elastics removed for clarity (when relaxed, the elastics typically cause the surrounding material to gather or "shirr"). In the flattened position, the garment 10 may have a generally hourglass shaped structure, but it may also have any other shape suitable for the given application, such as a rectangular shape, a trapezoidal shape, a "T" shape, and the like. As used herein, the longitudinal axis 100 of the garment is the dimension of the garment corresponding to the front-to-rear dimension of the user, and the lateral axis 102 of the garment is the dimension corresponding to the side-to-side dimension of the user.

In use, the invention comprises a pant-like garment 10 having a waist-encircling region and a crotch region. The waist-encircling region may comprise a first waist region 12, disposed adjacent to, for example, the back waist region of a wearer's body, and a second waist region 14, disposed adjacent to, for example, the front waist region of a wearer's body. The first and second waist regions 12,14, may correspond to the front and back of the wearer's body, respectively, depending on whether garment 10 is attached in front of or behind the subject wearer. The first and second waist regions are joined together at or near their lateral edges 18, causing the longitudinally distal edges 20 of the garment 10 to form the perimeter of a waist opening. A crotch region 16 extends between the first and second waist regions 12, 14, and the crotch edges 22 form the perimeter of a pair of leg openings, when the garment 10 is placed on a subject wearer.

The garment 10 preferably comprises a top sheet 24, and a back sheet 26, which may be substantially coterminous with the top sheet 24. When the garment 10 is being worn, the top sheet 24 faces the wearer's body, and the back sheet 26 faces away from the wearer. An absorbent laminate core 28 preferably is disposed between at least a portion of the top sheet 24 the back sheet 26.

An embodiment of the present invention may further comprise various additional features. One or more pairs of elastic gathers 30 may extend adjacent the crotch edges 22. The garment 10 may also comprise one or more waste containment systems, such as inboard standing leg gathers 40, which preferably extend from the second waist region 14 to the first waist region 12 along opposite sides of longitudinal center line 100 (only one standing leg gather system 40 is shown in Figure 1 for purposes of clarity). One or both of the first and second waist regions 12, 14 may also be equipped with strips of elastic waist foam 32 or other elastically extensible material, which help contract the garment around the wearer's waist, providing improved fit and leakage prevention.

The absorbent garment 10 also preferably includes fastening elements to enable attachment of the first waist region 12 to second waist region 14. Fastening elements preferably include a pair of tabs 34 that extend laterally away from opposite lateral edges 18 of the first waist region 12 of the garment 10. The tabs 34 may comprise an elastically extensible material (not shown), and may be designed to stretch around a wearer's waist to provide improved fit, comfort, and leakage protection. Such elasticized tabs 34 may be used in conjunction with, or in lieu of, waist foam 32, or other elastically extensible materials 32.

At least one fastening mechanism 36 (collectively referred to as "fastener 36") is attached to each tab 34 for attaching the tab to the second waist region 14, thereby providing the garment 10 with a pant-like shape, and enabling garment 10 to be fixed or otherwise fitted on the wearer. The fasteners 36 may attach to one or more target devices 38 located in the second waist region 14.

Although not shown in the drawings, the absorbent garment 10 may also include grips attached along one of its edges proximal to each tab 34 to enable a caregiver to pull the grips, and not on the ends of the tabs 34, around the wearer and over the target devices 38 to thereby secure the fasteners 36 to the one or more target devices 38.

The various parts of the garment 10 can be attached to one another or associated with one another to form a structure that preferably maintains its shape during the useful life of the garment 10. As used herein, the terms "attached," "joined," "associated," and similar terms encompass configurations whereby a first part is directly joined to a second part by affixing the first part directly to the second part, by indirectly joining the first part to the second part through intermediate members, and by fixing the relative positions of various parts by capturing parts between other parts. Those skilled in the art will appreciate that various methods or combinations of methods may be used to securely join the respective parts of the garment 10 to one another.

The top sheet 24 and back sheet 26 may be constructed from a wide variety of materials known in the art. The invention is not intended to be limited to any specific materials for these components. The top sheet 24 and back sheet can be shaped and sized according to the requirements of each of the various types of absorbent garment, or to accommodate various user sizes. In an embodiment of the invention in which the garment 10 is a diaper or an adult incontinence brief, the combination of top sheet 24 and back sheet 26, may have an hourglass shape, as seen in Figure 1, or may have a rectangular, trapezoidal, "T" shape, or other shape.

Due to the wide variety of backing and liner sheet construction and materials currently available, the invention is not intended to be limited to any specific materials or constructions of these components. The back sheet 26 preferably is made from any suitable pliable liquid-impervious material known in the art. Typical back sheet materials include films of polyethylene, polypropylene, polyester, nylon, and polyvinyl chloride and blends of these materials. For example, the back sheet can be made of a polyethylene film having a thickness in the range of 0.02-0.04 mm. The back sheet 26 may be pigmented with, for example, titanium dioxide, to provide the garment 10 with a pleasing color or to render the back sheet 26 opaque enough that exudates being contained by the garment 10 are not visible from outside the garment. In addition, the back sheet 26 may be formed in such a manner that it is opaque, for example, by using various inert components in the polymeric film and then biaxially stretching the film. Other back sheet materials will be readily apparent to those skilled in the art. The back sheet 26 preferably has sufficient liquid imperviousness to prevent any leakage of fluids. The required level of liquid imperviousness may vary between different locations on the garment 10.

The back sheet 26 may further comprise separate regions having different properties. In a preferred embodiment, portions of the back sheet 26 are air-permeable to improve the breathability, and therefore comfort, of the garment 10. The different regions may be formed by making the back sheet 26 a composite of different sheet materials, chemical treatment, heat treatment, or other processes or methods known in the art. Some regions of the back sheet 26 may be fluid pervious. In one embodiment of the invention, the back sheet 26 is fluid impervious in the crotch 16, but is fluid pervious in portions of the first and second waist regions 12,14. The back sheet 26 may also be made from a laminate of overlaid sheets of material.

The moisture-pervious top sheet 24 can be comprised of any suitable relatively liquid-pervious material known in the art that permits passage of liquid there through. Non-woven liner sheet materials are exemplary because such materials readily allow the passage of liquids to the underlying absorbent laminate core 28. Examples of suitable liner sheet materials include non-woven spun-bond or carded webs of polypropylene, polyethylene, nylon, polyester and blends of these materials.

The back sheet 26 may be covered with a fibrous, non-woven fabric such as is disclosed, for example, in U.S. Patent 4,646,362 issued to Heran et al*.,* the disclosure of which is hereby incorporated by reference in its entirety and in a manner consistent with this disclosure. Materials for such a fibrous outer liner include a spun-bonded non-woven web of synthetic fibers such as polypropylene, polyethylene or polyester fibers; a non-woven web of cellulosic fibers, textile fibers such as rayon fibers, cotton and the like, or a blend of cellulosic and textile fibers; a spun-bonded non-woven web of synthetic fibers such as polypropylene; polyethylene or polyester fibers mixed with cellulosic, pulp fibers, or textile fibers; or melt blown thermoplastic fibers, such as macro fibers or micro fibers of polypropylene, polyethylene, polyester or other thermoplastic materials or mixtures of such thermoplastic macro fibers or micro fibers with cellulosic, pulp or textile fibers. Alternatively, the back sheet 26 may comprise three panels wherein a central poly back sheet panel is positioned closest to absorbent laminate core 28 while outboard non-woven breathable side back sheet panels are attached to the side edges of the central poly back sheet panel. Alternatively, the back sheet 26 may be formed from microporous poly coverstock for added breathability.

As illustrated in more detail in Figure 2, the top sheet 24 may be formed of three separate portions or panels. Those skilled in the art will recognize, however, that top sheet 24 need not be made of three separate panels, and that it may be comprised of one unitary item. A first top sheet panel 301 may comprise a central top sheet panel formed from preferably a liquid-pervious material that is either hydrophobic or hydrophilic. The central top sheet panel 301 may be made from any number of materials, including synthetic fibers (e.g., polypropylene or polyester fibers), natural fibers (e.g., wood or cellulose), apertured plastic films, reticulated foams and porous foams to name a few. One preferred material for a central top sheet panel 301 is a cover stock of single ply non-woven material which may be made of carded fibers, either adhesively or thermally bonded, perforated plastic film, spunbonded fibers, or water entangled fibers, which generally weigh from 0.3-0.7 oz./sq. yd. and have appropriate and effective machine direction and cross-machine direction strength suitable for use as a baby diaper cover stock material. The central top sheet 301 panel preferably extends from substantially the second waist region 14 to the first waist region 12, or a portion thereof.

The second and third top sheet panels 302, 303 (e.g., outer top sheet panels), in this alternative embodiment may be positioned laterally outside of the central top sheet panel 301. The outer top sheet panels 302, 303 are preferably substantially liquid-impervious and hydrophobic, preferably at least in the crotch area. The outer edges of the outer top sheet panels may substantially follow the corresponding outer perimeter of the back sheet 26. The material for the outer top sheet portions or panels is preferably polypropylene and can be woven, non-woven, spunbonded, carded or the like, depending on the application.

The inner edges 304 (FIG. 2) of the outer top sheet portions or panels 302,303 preferably are attached by, e.g., an adhesive, to the outer edges 305 of the inner top sheet portion or panel 301. At the point of connection with the outer edges 305 of the inner top sheet portion or panel 301, the inner edges 304 of the outer top sheet portions or panels 302, 303 extend upwardly to form waste containment flaps 40. The waste containment flaps 40 preferably are formed of the same material as the outer top sheet portions or panels 302, 303, as in the embodiment shown. They are preferably an extension of the outer top sheet portions or panels 302, 303.

The waste containment flaps 40 may be treated with a suitable surfactant to modify their hydrophobicity/hydrophilicity as desired, and they may be treated with skin wellness ingredients to reduce skin irritation. Alternatively, the waste containment flaps 40 may be formed as separate elements and then attached to the body side liner. In this alternative embodiment, the central top sheet portion or panel 301 may extend past the connection point with the waste containment flaps 40, and even extend to the periphery of the back sheet 26.

The waste containment flaps 40 preferably include a portion that folds over onto itself to form a small enclosure. At least one, and depending on the size of the enclosure sometimes more than one, elastic member 42 may be secured in the enclosure in a stretched condition. As is known in the art, when the flap elastic 42 attempts to assume the relaxed, unstretched condition, the waste containment flaps 40 rise above the surface of the central top sheet portion or panel 301.

The top sheet 24 (as well as top sheet portions 301, 302, 303) may be made of any suitable relatively liquid-pervious material currently known in the art or later discovered that permits passage of a liquid there through. Examples of suitable top sheet materials include non-woven spun-bonded or carded webs of polypropylene, polyethylene, nylon, polyester and blends of these materials, perforated, apertured, or reticulated films, and the like. Non-woven materials are exemplary because such materials readily allow the passage of liquids to the underlying absorbent laminate core 28. The top sheet 24 preferably comprises a single-ply non-woven material that may be made of carded fibers, either adhesively or thermally bonded, spun-bonded fibers, or water entangled fibers, which generally weigh from 0.3 - 0.7 oz./sq. yd. and have appropriate and effective machine direction (longitudinal) and cross-machine (lateral) direction strength suitable for use as a top sheet material for the given application. The present invention is not intended to be limited to any particular material for the top sheet 24, and other top sheet materials will be readily apparent to those skilled in the art.

The top sheet 24 may further comprise several regions having different properties. In one embodiment of the present invention, the laterally distal portions of the top sheet 24, especially those used to make second and third top sheet panels 302, 303, preferably are substantially fluid impervious and hydrophobic, while the remainder of the top sheet 24 (e.g., central top sheet panel 301) is hydrophilic and fluid pervious. Different top sheet properties, such as fluid perviousness and hydrophobicity, may be imparted upon the top sheet 24 by treating the top sheet 24 with adhesives, surfactants, or other chemicals, using a composite of different materials, or by other means. The top sheet 24 may also be made from a laminate of overlaid sheets of material. The top sheet 24 also may be treated in specific areas like the crotch region, with skin wellness ingredients such as aloe, vitamin E, and the like.

As noted elsewhere herein, the top sheet 24 and back sheet 26 may be substantially coterminous, or they may have different shapes and sizes. The particular design of the top sheet 24 and back sheet 26 may be dictated by manufacturing considerations, cost considerations, and performance considerations. Preferably, the top sheet 24 is large enough to completely cover the absorbent laminate core 28, and the back sheet 26 is large enough to prevent leakage from the garment 10. The design of top sheet 24 and back sheet 26 is known in the art, and a skilled artisan will be able to produce an appropriate top sheet 24 and an appropriate back sheet 26 without undue experimentation.

The top sheet 24 and the back sheet 26 may be associated with one another using a variety of methods known in the art. For example, they may be thermally, ultrasonically, or chemically bonded to one another. They also may be joined using lines of hot melt adhesive or mechanical fasteners, such as thread, clips, or staples. In one embodiment, a hydrophilic adhesive, such as Cycloflex as sold by National Starch, a corporation headquartered in Bridgewater, New Jersey, is used to join the top sheet 24 to the back sheet 26. The particular joining method may be dictated by the types of materials selected for the top sheet 24 and back sheet 26.

As mentioned above, absorbent garment preferably is provided with leg elastics 30 extending through crotch region 16, adjacent crotch edge 22. The absorbent garment of the invention also preferably is provided with waist elastic material 32 optionally in the first and second waist regions, 12, 14, respectively, to enable and assist in stretching around the wearer. The waist elastics 32 may be similar structures or different to impart similar or different elastic characteristics to the first and second waist regions 12,14 of the garment. In general, the waist elastics may preferably comprise foam strips positioned at the first and second waist regions 12,14, respectively. Such foam strips preferably are about ½ to about 1½ inches wide and about 3-6 inches long. The foam strips preferably are positioned between the top sheet portions 24 or panels (301, 302, 303) and the back sheet 26. Alternatively, a plurality of elastic strands may be employed as waist elastics rather than foam strips. The foam strips preferably are comprised of polyurethane, but can be any other suitable material that decreases waist band roll over, reduces leakage over the waist ends of the absorbent garment, and generally improve comfort and fit. The first and optional second waist foam strips 32 preferably are stretched 50-150%, preferably 100% more than their unstretched dimension before being adhesively secured between the back sheet 26 and top sheet 24.

Each edge 22 that forms the leg openings preferably is provided with an adjacent leg elastic containment system 30. In the preferred embodiment, three strands of elastic threads (only two strands are shown in Figure 2 for purposes of clarity) are positioned to extend adjacent to leg openings between the outer top sheet portions or panels 302, 303 and the back sheet 26. Any suitable elastomeric material exhibiting at least an elongation (defined herein as (Lₛ -L_{R})/L_{R} where Lₛ is the stretch length of an elastic element and L_{R} is retracted length, multiplied by 100 to obtain percent elongation) in the range of 5%-350%, preferably in the range of 200%-300%, can be employed for the leg elastics 30. The leg elastics 30 may be attached to the absorbent article 10 in any of several ways which are known in the art. For example, the leg elastics 30 may be ultrasonically bonded, heat/ pressure sealed using a variety of bonding patterns, or glued to the garment 10. Various commercially available materials can be used for the leg elastics 30, such as natural rubber, butyl rubber or other synthetic rubber, urethane, elastomeric materials such as LYCRA (DuPont), GLOSPAN (Globe) or SYSTEM 7000 (Fulflex).

The fastening elements, preferably a fastening system 34 (e.g., tab 34) of the preferred embodiment, is attached to the first waist region 12, and it preferably comprises a tape tab or mechanical fasteners 36. However, any fastening mechanism known in the art will be acceptable. Moreover, the fastening system 34 may include a reinforcement patch below the front waist portion so that the diaper may be checked for soiling without compromising the ability to reuse the fastener. Alternatively, other absorbent article fastening systems are also possible, including safety pins, buttons, and snaps.

As stated previously, the invention has been described in connection with a diaper. The invention, however, is not intended to be limited to application only in diapers. Specifically, the absorbent laminate cores of the preferred embodiments may be readily adapted for use in other absorbent garments besides diapers, including, but not limited to, training pants, feminine hygiene products and adult incontinence products.

The underlying structure beneath the top sheet 24 may include, depending on the diaper construction, various combinations of elements, but in each embodiment, it is contemplated that the absorbent garment will include an absorbent laminate core 28 comprising more than one laminates positioned between the top sheet 24 and back sheet 26. The absorbent laminate core 28 may take a number of different constructions, depending on how the laminates are configured with respect to one another. However, in each embodiment, one of the laminated layers in the absorbent laminate core 28 is a central fibrous layer containing tow fibers and from 30 to 95% by weight SAP, and preferably, greater than 50% by weight SAP.

With particular reference to FIG. 2, and FIGs. 3-15, there are illustrated exploded partial cross-sectional schematic views of absorbent laminate cores 28 according to various preferred embodiments of the invention. Although the absorbent laminate core 28 depicted in the accompanying figures has a substantially rectangular cross-sectional and plan view shape, other shapes may be used, such as a "T" shape or an hourglass shape. The shape of the absorbent laminate core 28 may be selected to provide the greatest absorbency with a reduced amount of material. The absorbent laminate core may be associated with the top sheet 24, back sheet 26, or any other suitable part of the garment 10 by any method known in the art, in order to fix the absorbent laminate core 28 in place. In addition to the respective layers in the absorbent laminate core 28, as will be described in greater detail hereinafter, the overall absorbent laminate core 28 may be enclosed within a tissue wrapping. Skilled artisans are capable of designing and wrapping a suitable absorbent laminate core 28 of the invention, using the guidelines provided herein.

The absorbent laminate core 28 may extend into either or both of the first and second waist regions 12, 14. The absorbent laminate core 28 of one preferred embodiment of the invention includes at least four (4) layers whereby two of the layers are outer layers, (280, 282, Fig. 3) and one of the inner layers is a central fibrous layer 284 containing more than 50% by weight SAP. The absorbent laminate core 28 further includes as the at least one other inner or central layer 286, additional layer selected from a fluid acquisition layer, a distribution layer, a wicking layer, or combinations and fragments of these layers. The at least two inner layers, the central fibrous layer 284 containing more than 50% by weight SAP, and the at least one other inner layer 286, are disposed between the upper layer 280 and the lower layer 282.

Upper layer 280 and lower layer 282 can be made of any suitable material capable of containing the inner layers (284, 286, 288, 286', etc.) of absorbent laminate core 28. Preferably, upper layer 280 is hydrophilic and fluid pervious, and lower layer 282 is hydrophobic and fluid impervious. More preferably, upper layer 280 and lower layer 282 are comprised of the same tissue-like material.

In a preferred embodiment, the central fibrous layer 284 of absorbent laminate core 28 comprises super absorbent polymer distributed within a fibrous structure. Central fibrous layers 284 of this type are known in the art, and exemplary absorbent cores are described in U.S. Pat. No. 6,068,620 and U.S. Pat. No. 5,281,207, both issued to Chmielewski, and U.S. Pat. No. 5,863,288, issued to Baker, the disclosures of each of which are herein incorporated by reference in their entirety and in a manner consistent with this disclosure.

Certain fibrous and particulate additives preferably are used as constituent elements of an absorbent core laminate. Super absorbent polymers of the surface cross-linked variety perform best in these laminates. These additives preferably are constituent elements of the central fibrous layer 284, and they may be added to the additional layer 286 (and optional layers 288). Fibrous additives of central fibrous layer 284 preferably include, but are not limited to, cellulose acetate fibers, rayon fibers, Courtauld's LYOCELL fibers, polyacrylonitrile fibers, surface-modified (hydrophilic) polyester fibers, surface-modified polyolefin/ polyester bicomponent fibers, surface-modified polyester/polyester bicomponent fibers, cotton fibers, or blends thereof. Of the foregoing, cellulose acetate is the most preferred fibrous additive for use in central fibrous layer 284. In addition, rayon, Courtauld's LYOCELL, polyacrylonitrile, cotton fibers and cotton linters have similar properties to cellulose acetate and are alternatively preferred. The remaining fibers, surface-modified polyolefin/ polyester bicomponent fibers, and surface-modified polyester/ polyester bicomponent fibers are also believed to be effective fibrous additives. The concentration of fibrous additives in the central fibrous layer 284 of the absorbent laminate core 28 of the invention preferably is about 5-50%, more preferably about 10-30%, and most preferably about 15-25%. Most preferably, the central fibrous layer 284 comprises from about 75-85% SAP and from about 15-25% fibrous additives selected from the foregoing group.

The fibrous component of the central fibrous layer 284 most preferably is a crimped tow of cellulose acetate or polyester.

Optionally, it is advantageous to introduce from about 1-5% of a thermally bondable fiber into the fibrous component of the central fibrous layer 284 for wet strength and core stability in use.

Particulate additives may be added to central fibrous layer 284 in addition to or as a substitute for the foregoing fibrous additives in order to maintain high SAP efficiency. The particulate additives preferably are insoluble, hydrophilic polymers with particle diameters of 100 pm or less. The particulate additives are chosen to impart optimal separation of the SAP particles. Examples of preferred particulate additive materials include, but are not limited to, potato, corn, wheat, and rice starches. Partially cooked or chemically modified (*i.e*., modifying hydrophobicity, hydrophilicity, softness, and hardness) starches can also be effective. Most preferably, the particulate additives comprise partially cooked corn or wheat starch because in this state, the corn or wheat are rendered larger than uncooked starch and even in the cooked state remain harder than even swollen SAP. In any event, regardless of the particulate additive chosen, one of the many important criteria is to use particulate additives that are hard hydrophilic materials relative to swollen SAP or which are organic or inorganic polymeric materials about 100 microns in diameter. Fibrous and particulate additives can be used together in these absorbent laminates. Examples of SAP/particulate and SAP/fiber/particulate additives include those described in, for example, U.S. Patent No. 6,068,620.

Any superabsorbent polymer (SAP) now known or later discovered may be used in central fibrous layer 284, so long as it is capable of absorbing liquids. Useful SAP materials are those that generally are water-insoluble but water-swellable polymeric substance capable of absorbing water in an amount that is at least ten times the weight of the substance in its dry form. In one type of SAP, the particles or fibers may be described chemically as having a back bone of natural or synthetic polymers with hydrophilic groups or polymers containing hydrophilic groups being chemically bonded to the back bone or in intimate admixture therewith. Included in this class of materials are such modified polymers as sodium neutralized cross-linked polyacrylates and polysaccharides including, for example, cellulose and starch and regenerated cellulose which are modified to be carboxylated, phosphonoalkylated, sulphoxylated or phosphorylated, causing the SAP to be highly hydrophilic. Such modified polymers may also be cross-linked to reduce their water-solubility.

Commercially available SAPs include a starch modified superabsorbent polymer available under the trade name SANWET® from Hoechst Celanese Corporation, Portsmouth, VA. SANWET® is a starch grafted polyacrylate sodium salt. Other commercially available SAPs include a superabsorbent derived from polypropenoic acid, available under the tradename DRYTECH® 520 SUPERABSORBENT POLYMER from The Dow Chemical Company, Midland Mich.; AQUA KEEP manufactured by Seitetsu Kagaku Co., Ltd.; ARASORB manufactured by Arakawa Chemical (U.S.A.) Inc.; ARIDALL 1125 manufactured by Chemdall Corporation; FAVOR and SP-1365, manufactured by Stockhausen Inc.; HYSORB and P-7710, manufactured by BASF, Ludwigshafen, Germany; AQUA KEEP SA60S, manufactured by Seitetsu Kagaku Co., Ltd.; DIAWET, commercially available from Mitsubishi Chemicals, Japan; FLOSORB, available from SNF Floerger, France; SA 55 SX produced by Sumitomo; AQUALIC, available from Nippon Shokubai, Osaka, Japan.

In accordance with the present invention, the absorbent composite core comprises tow fibers and SAP. The tow fiber preferably is a continuous crimped filament tow. This fiber structure has high structural integrity, and as such, is distinct from a matrix of discontinuous fibers described as fluff in the prior art. The high structural integrity enables the production of stronger webs than those formed from discontinuous fibers, which in turn are believed to enable the production of thinner absorbent pads. In addition, the use of such fibers enables the production of ultra low density absorbent cores, when compared to absorbent cores prepared by dispersing SAP particles in fluff.

The tow fiber can be any continuous or discontinuous thermoplastic filament tow fiber that is capable of being opened and used in combination with SAP in an absorbent core. Preferably, cellulose ester tow is used as the fibrous material in central layer 284. Non-limiting examples of suitable cellulose esters include cellulose acetate, cellulose propionate, cellulose butyrate, cellulose caproate, cellulose caprylate, cellulose stearate, highly acetylated derivatives thereof such as cellulose diacetate, cellulose triacetate and cellulose tricaproate, and mixtures thereof such as cellulose acetate butyrate. A suitable cellulose ester will include the ability to absorb moisture, preferably is biodegradable, and is influenced not only by the substituent groups but also by the degree of substitution. The relationship between substituent groups, degree of substitution and biodegradability is discussed in W. G. Glasser et al, BIOTECHNOLOGY PROGRESS, vol. 10, pp. 214-219 (1994), the disclosure of which is incorporated herein by reference in its entirety.

Continuous filament tow useful in the present invention is beneficially moisture-absorbent and biodegradable. Accordingly, cellulose acetate tow is typically preferred for use in the invention. Typically, the denier per fiber (dpf) of the tow fiber will be in the range of about 1 to 9, preferably about 3 to 6. For the same weight product, filaments of lower dpf may provide increased surface area and increased moisture absorption. Total denier may vary within the range of about 20,000 to 60,000, depending upon the process used.

It is particularly preferred in the invention to use tow having crimped filaments. Tow materials having crimped filaments are typically easier to open. Separation of filaments resulting from bloom advantageously results in increased available filament surface area for superabsorbent material immobilization and increased moisture absorption. Gel blocking also may be reduced by using crimped tow in the central layer 284. As therefore may be understood, more crimp is typically better, with in excess of about 20 crimps per inch being usually preferred. Continuous filament, cellulose ester tow having crimped filaments with about 25 to 40 crimps per inch, is commercially available from Celanese Acetate in Charlotte, N.C.

The total basis weights of the absorbent laminate core 28 including fibrous materials, SAP, tissue, additional layers, and additives, are anywhere from about 200-1,000 grams per square meter. The most preferred total basis weights of the absorbent laminate core 28 are about 400-700 grams per square meter, and even more preferably from about 250 to about 350 grams per square meter. The foregoing fibrous additives maintain high SAP efficiency at high SAP concentrations even when they are mixed with soft or hard wood fluff pulp fibers.

Optionally, about 1-10%, preferably about 5%, by weight of thermally bondable synthetic fibers can be added to the absorbent laminate core 28 to impart additional wet strength to the laminate. This will improve the stability of the core during use of the diaper. The preferred synthetic fibers are polyolefin/ polyester fibers and polyester/polyester bicomponent fibers.

While, as discussed above, the present invention is premised in part on the discovery that certain fibrous and particulate additives maintain high SAP efficiencies when the SAP concentration is in the range of about 30-95%, preferably, 50-95% fluff/SAP, central fibrous layers 284 that contain greater than about 50% SAP may require additional structural or design measures to contain the SAP in the layer and provide adequate wet strength for overall core stability in manufacture and use. One solution is to adhesively or thermally bond the absorbent material to improve wet strength and core stability. This unfortunately results in slower than adequate rates of absorption and poor SAP efficiency. Another solution resides in the discovery that a high SAP concentration central fibrous layer 284 may be hydrogen bonded to additional fibrous layers. When a highly concentrated SAP-containing central fibrous layer 284 is hydrogen bonded to additional layer 286, and/or to upper and lower layers 280, 282, or optionally is wrapped by a fibrous layer (not shown), the SAP efficiency is not impaired, wet strength increases, and the upper and lower layers 280, 282, and optional wrapping layer add stability to the core during manufacture. The structure and composition of the absorbent laminate cores 28 preferably are designed for optimal strength, SAP containment, and liquid distribution. Skilled artisans are capable of designing absorbent laminate cores 28 to optimize the foregoing attributes, using the guidelines provided herein.

Depending on whether a wet or dry process is used to make the absorbent laminate cores 28, bonding central fibrous layer 284 with additional layer 286, and tissue layers 280, 282, can be achieved with hydrogen or adhesive bonds. If the material used to form the absorbent laminate cores 28 contains about 1-5% by weight thermally bondable synthetic fibers, bonding can be achieved with thermal bonds. When the upper and lower layers 280, 282 are tissue layers and are hydrogen bonded using water to middle layers 284, 286 (and optionally 288), unexpectedly good "core utilization" is realized. "Core utilization" is the percentage of the total capacity of a core that can be absorbed in a demand absorbency test. This unexpected performance improvement is believed to be the result of the good liquid distribution achieved with a high density, non-gel blocking central fibrous layer 284, at least one additional layer 286, and using tissue layers 280, 282 that are intimately bonded to the fibers of the inner layers 284, 286 of the absorbent laminate core 28.

The absorbent laminate cores 28 of the invention include two outer layers 280, 282, a central fibrous layer 284, and at least one additional layer 286. The at least one additional layer can be any layer selected from a fluid acquisition layer, a distribution layer, a wicking layer, or combinations and fragments of these layers. Such layers may be provided to assist with transferring fluids to the absorbent core 28, handling fluid surges, preventing rewet, containing absorbent material, improving core stability, or for other purposes. Skilled artisans are familiar with the various additional layers that may be included in absorbent article, and the present invention is not intended on being limited to any particular type of materials used for those layers. Rather, the invention encompasses all types of wicking layers, all types of distribution layers, etc., to the extent that type of layer is utilized.

One element that is useful as an additional layer 286 in the absorbent laminate core 28 of the invention is a fluid acquisition layer. The fluid acquisition layer typically comprises a hydrophilic fibrous material, and serves to quickly collect and temporarily hold discharged body fluid. A portion of discharged fluid may, depending upon the wearer's position, permeate the acquisition layer and be absorbed by the central fibrous layer 284 in the area proximate to the discharge. However, since fluid is frequently discharged in gushes, the central fibrous layer 284 in such area may not absorb the fluid as quickly as it is discharged. Therefore, the fluid acquisition layer 286 hereof also facilitates transport of the fluid from the point of initial fluid contact to other parts of the absorbent laminate composite 28. In the context of the present invention, it should be noted that the term "fluid" includes, but is not limited to, liquids, urine, menses, perspiration, and water based body fluids.

The function of the fluid acquisition layer 286 is relatively important. The fluid acquisition layer 286 preferably has sufficient capillary suction to more fully drain the top sheet 24 and yet not exhibit excessive fluid retention to make it difficult for the underlying layer (e.g., central fibrous layer 284) to desorb the acquisition layer 286. The acquisition layer 286 may be comprised of several different materials including nonwoven or woven webs of synthetic fibers including polyester, polypropylene, or polyethylene, natural fibers including cotton or cellulose, blends of such fibers, foams, fluff pulp, apertured films, or any equivalent materials or combinations of materials.

Another useful layer for use in the absorbent laminate core 28 of the invention includes a fluid distribution layer 286. Fluid distribution layers 286 of the invention can include any combination or all of three basic components: chemically stiffened, twisted, and curled bulking fibers, high surface area fibers, and binder fibers. In a preferred embodiment of the invention, fluid distribution layer 286 comprises from about 20% to about 80% of the chemically stiffened, twisted, and cured fibers, from about 10% to about 80% of a high surface area fiber, and from 0% to about 50% of a thermoplastic binding means for increasing physical integrity of the web. All percentages herein refer to weight percentages based on total dry web weight. Preferably, the fluid distribution layer 286 will comprise between about 45% and about 60% of chemically stiffened, twisted, and cured fibers, between about 5% and about 15% of a hot melt fibrous binding means, and between about 30% and about 45% high surface area cellulose binding means. More preferably, the fluid distribution layer 286 comprises about 10% thermoplastic binding means, about 45% chemically stiffened, twisted, and cured fibers, and about 45% high surface area fibers.

Chemical additives can also be used as binding means, and are incorporated into the acquisition/distribution layer at levels typically of about 0.2% to about 2.0%, dry web weight basis. The three basic fiber components are described in greater detail in U.S. Patent No. 5,549,589, the disclosure of which is incorporated by reference herein in its entirety, and in a manner consistent with this disclosure.

Fluid distribution layer 286 also may be comprised of non-woven or woven webs of synthetic fibers, natural fibers, foams, carded, thermal bonded materials, and the like. Another useful layer in absorbent laminate core 28 is a wicking layer 286. Wicking layers usually have both fluid acquisition and fluid distribution properties. For example, vertical wicking, which is in general the ability to transport fluids vertically from the top sheet 24 to the absorbent laminate core 28, is related in many respects to fluid acquisition. Horizontal wicking, which is in general the ability to transport fluids along the horizontal 100 and vertical 102 axes of Figure 1, is related in many respects to fluid distribution.

Any conventional wicking materials can be used for the wicking layer 286 of the invention. High internal phase emulsion (HIPE) foams such as those disclosed in U.S. Patent No. 5,650,222 can be used, braided materials such as those disclosed in H1,585, and other conventional fibrous and strand materials can be used. The disclosures of U.S. Patent No. 5,650,222 and H1,585 are incorporated by reference here in their entirety, and in a manner consistent with the present invention.

Wicking layer 286 also may be comprised of two or more sublayers containing absorbent materials with differing wicking characteristics. Any of the materials discussed in this context can be used for any and all of the wicking layers 286. In accordance with the embodiment of the invention discussed immediately above, the wicking layer 286 may include a first member that is made of a material that is capable of rapidly transferring, in the z-direction (e.g., orthogonal to the plane formed by horizontal 100 and vertical 102 axes of Figure 1), body fluid that is delivered to top sheet 24. The first member may be designed to have a dimension narrower than the dimension of the absorbent laminate core 28. In this regard, the sides of the first member preferably are spaced away from the longitudinal sides of the absorbent laminate core 28 so that body fluid is restricted to the area within the periphery of the first member, before it passes down and is absorbed into central fibrous layer 284 (or second member of the wicking layer 286). This design is believed to enable the body fluid to be combined in the central area of the absorbent laminate core 28 and to be wicked downward so that a greater quantity of the central fibrous layer 284 can be utilized.

A suitable material for use as a first member having high wicking capacity in the z-direction, is a material available from Kimberly-Clark Corporation, in Neenah, Wis. known as PRISM. PRISM is described in U.S. Pat. No. 5,336,552, which is hereby incorporated by reference in its entirety, and in a manner consistent with this disclosure. PRISM generally is a nonwoven fabric and comprises extruded multicomponent polymeric strands including first and second polymeric components arranged in substantially distinctive zones across the cross-section of the multicomponent strands and extending continuously along the length of the multicomponent strands. Preferably, the strands are continuous filaments which may be formed by spunbonding techniques. The second component of the strands constitutes at least a portion of the peripheral surface of the multicomponent strands continuously along the length of the multicomponent strands and includes a blend of a polyolefin and an ethylene alkyl acrylate copolymer. Bonds between the multicomponent strands may be formed by the application of heat.

More specifically, the first polymeric component of the multicomponent strands is present in an amount of from about 20 to about 80 percent by weight of the strands, and the second polymeric component is present in an amount from about 80 to about 20 percent by weight of the strands. Preferably, the first polymeric component of the multicomponent strands is present in an amount of from about 40 to about 60 percent by weight of the strands and the second polymeric component is present in an amount from about 60 to about 40 percent by weight of the strands.

The term "strand" as used herein refers to an elongated extrudate formed by passing a polymer through a forming orifice such as a die. Strands include fibers, which are discontinuous strands having a definite length, and filaments, which are continuous strands of material. The non-woven fabric of the present invention may be formed from staple multicomponent fibers. Such staple fibers may be carded and bonded to form the non-woven fabric. Preferably, however, the non-woven fabric of the present invention is made with continuous spunbond multicomponent filaments which are extruded, drawn and laid on a traveling forming surface.

The types of non-woven materials that may be employed in any of the wicking layers 286 of the invention include powder-bonded-carded webs, infrared bonded carded webs, and through-air-bonded-carded webs. The infrared and through-air bonded carded webs can optionally include a mixture of different fibers, and the fiber lengths within a selected fabric web may be within the range of about 1.0 to 3.0 inch and an average bulk density of about 0.02 g/ cc to about 0.06 g/cc.

The first member of wicking layer 286 also may be a non-woven fibrous web which includes about 75 percent polyester fibers of at least 6 denier, such as PET (polyethylene terephthalate) fibers available from Celanese AG. The polyester fibers have a length ranging from about 1.5 to 2.0 inches in length. The remaining 25 percent of the fibrous web can be composed of bicomponent binder fibers of not more than 3 denier, and preferably about 1.5 denier. The bicomponent fiber length ranges from about 1.5 to 2 inches. Suitable bicomponent fibers are wettable, polyethylene/ polypropylene bicomponent fiber, available from Chisso, a business having offices located in Osaka, Japan. The bicomponent fiber can be a composite, sheath-core type with the polypropylene forming the core and polyethylene forming the sheath of the composite fiber. The polyester fibers and bicomponent fibers generally are homogeneously blended together and are not in a layered configuration. The fibers can be formed into a carded web which is thermally bonded, such as by through-air bonding or infrared bonding.

The second member of wicking layer 286 may be positioned vertically below the first member, and it preferably has a higher wicking capacity along the longitudinal 100 and vertical 102 axes of Figure 1, than the first member. Preferably, the second member has a wicking capacity at least three time greater than the first member. The second member can be equal in width to the first member, but preferably will be wider. It is preferred that the width of the wicking layer 286 in general be the same as or greater than the width of central fibrous layer 284.

The second member can be a hydrophilic material formed from various types of natural or synthetic fibers including cellulose fibers, surfactant treated meltblown fibers, wood pulp fibers, regenerated cellulose, cotton fibers or a blend of other fibers. Preferably, the second absorbent member is a material described in U.S. Pat. No. 4,100,324, and is generally known as coform. Coform is available from the Kimberly-Clark Corporation located in Neenah, Wis. and is generally a non-woven material having a fabric-like finish and is made up of an airform matrix of thermoplastic polymeric fibers and a multiplicity of individualized wood pulp fibers. The thermoplastic fiber polymers generally have an average diameter of less than 10 microns with the individualized wood pulp fibers dispersed throughout the matrix and serving to space these microfibers from each other. The material is formed by initially utilizing the primary air stream with the meltblown microfibers and the secondary air stream containing wood pulp fibers and merging the two under turbulent conditions to form an integrated air stream along a forming surface. The fiber-like appearance of this material provides a visual appealing absorbent. Also inherent in the coform material is increased resiliency compared to conventional cellulosic absorbents.

Other suitable materials for use as wicking layer 286 include high-density air laid fluff pulps, high-density wet laid fluff pulp, and multi-groove fibers such as 4DG deep groove fiber, available from Eastman Chemical Company, Tennessee, or Clemson University, South Carolina

Various combinations of layers also may be used as the at least one additional layer 286. For example, additional layer 286 may be comprised of a combination of a wicking layer and a distribution layer. Hence, the additional layer 286 will have both wicking and distribution properties. The at least one additional layer also may be fragmented either along longitudinal axis 100 or vertical axis 102 to maximize the ability of absorbent laminate core 28 to rapidly absorb and retain fluids. Skilled artisans will be capable of designing additional layers 286 to have desired properties by combining various layer attributes, or by fragmenting the layer, using the guidelines provided herein.

The dimensions of additional layer(s) 286 may be the same as or different from the dimensions of central fibrous layer 284 and/or upper layer 280 and lower layer 282. It is preferred that additional layer(s) 286 have a width in the lateral direction (102) of anywhere from about 10 mm to about 100 mm, and preferably from about 25 mm to about 80 mm.

The foregoing absorbent laminate cores 28 of the preferred embodiments may be made using both wet and dry process, as described, for example, in U.S. Patent No. 6,068,620, the disclosure of which is incorporated by reference herein in its entirety. Skilled artisans are capable of making the absorbent laminate cores 28 of the present invention, using the guidelines provided herein.

Various preferred absorbent laminate core 28 configurations are shown in Figures 2-15. In Figure 2, the absorbent laminate core 28 is comprised of two outer layers, upper layer 280, and lower layer 282, which preferably are tissue layers. Disposed adjacent upper layer 280 preferably is central fibrous layer 284, which contains greater than 50% by weight SAP, and disposed between central fibrous layer 284 and lower layer 282, is the at least one additional layer 286. It is preferred in the embodiment shown in Figure 2, that the additional layer 286 be a wicking layer, although any of the additional layers described above could be used as additional layer 286.

The absorbent laminate core 28 of Figure 3 is essentially the inverse of that shown in Figure 2. Here, the additional layer 286 is disposed between the upper layer 280 and central fibrous layer 284. In this embodiment, additional layer 286 preferably is a fluid acquisition layer or a combination of a wicking and distribution layer (e.g., a layer having both wicking and distribution properties such as the multi-member wicking layer discussed above).

The absorbent laminate core 28 of the invention also may include another central fibrous layer 284 disposed between the additional layer 286 and lower layer 282. When more than one central fibrous layer 284 is used, different types of SAP may be used in each respective layer, or only one layer may contain SAP and the other be comprised of fibrous material. For example, central fibrous layer 284 that is disposed closest upper layer 280 may include a rapid acquisition SAP that has relatively low absorbence, whereas the central fibrous layer 284 disposed closest to lower layer 282 may include slower acquisition SAP with higher absorbency. This configuration would allow liquid to be rapidly absorbed, and then transferred to storage on the bottom of absorbent laminate core 28. Skilled artisans are capable of designing and fabricating the various central fibrous layers 284 when more than one of such layers are used, using the guidelines provided herein. The statements made herein refer to all of the embodiments of the invention that include two separate central fibrous layers 284 in absorbent laminate core 28, such as those illustrated in Figures 4, 5, 7, 8, and 11.

The preferred embodiment illustrated in Figure 4 includes in addition to the two central fibrous layers 284, an additional layer 286. It is preferred that additional layer 286 be a combination of a wicking and distribution layer (e.g., a layer having both wicking and distribution properties such as the multi-member wicking layer discussed above).

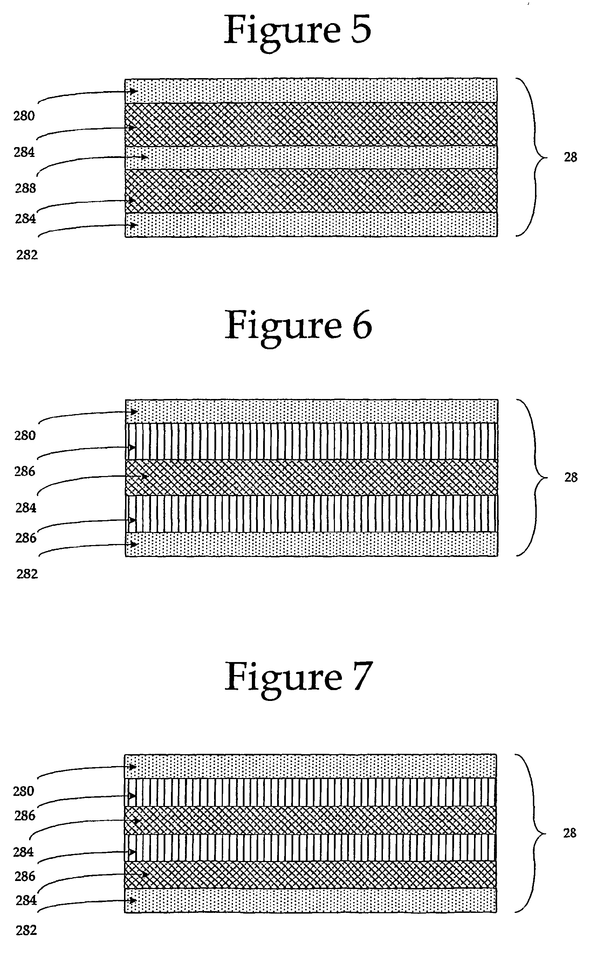

The absorbent laminate core 28 shown in Figure 5 includes two central fibrous layers 284 disposed between upper layer 280 and lower layer 282 (again, upper and lower layers 280, 282, preferably are tissue layers). The two central fibrous layer 284 are separated from one another by an optional additional layer 288. Preferably, optional additional layer 288 is a tissue layer, although it may be comprised of any of the materials used as additional layer 286, including another fibrous layer 284. In this embodiment, the respective central fibrous layers 284 may contain varying degrees of SAP, or one can contain more than 50% by weight SAP and the others contain no SAP at all.

Figure 6 illustrates a preferred embodiment whereby the central fibrous layer 284 is surrounded by two additional layer 286. In this embodiment, it is preferred that the additional layer 286 disposed between central fibrous layer 284 and upper layer 280 is a fluid acquisition layer, or a combination of a wicking and distribution layer. It also is preferred in this embodiment that the additional layer 286 disposed between central fibrous layer 284 and lower layer 282 be a combination of a wicking and distribution layer, or a storage layer.

Such storage layers 286 typically have limited transport and wicking capabilities but high storage or retention capacity, and rely upon the central fibrous layer 284 to distribute incoming fluid over a larger area.

Storage layers or members 286 may be of generally conventional design and composition, selected with regard to the particular application. The storage layer or member 286 may be monolayer or multilayer, homogeneous or stratified, profiled or uniform, etc. Materials suitable for use in such storage layers 286 may be natural or synthetic in origin, woven, non-woven, fibrous, cellular, or particulate, and may include particles, layers, or regions of absorbent polymeric gelling materials. Other preferred materials include fluff pulp and SAP composites, either air laid or wet laid, and high capacity resilient foam materials. Storage layer 286 may also have any desired size and/or shape as may prove suitable for a particular application, including square, rectangular, oval, elliptical, oblong, etc. They may also take on a three-dimensional shape or may be substantially planar in nature.

Figure 7 illustrates a preferred embodiment whereby alternating additional layer 286 and central fibrous layer 284 arrangement is used. The embodiment shown in Figure 7 illustrates one repeated alternating layer arrangement, although those skilled in the art recognize that more alternating layer arrangements may be employed. As shown therein, the absorbent laminate core 28 preferably comprises an upper layer 280, and in descending order below the upper layer 280, a first additional layer 286, a first central fibrous layer 284, a second additional layer 286, a second central fibrous layer 284, and lower layer 282. Preferably, the first additional layer 286 is selected from an acquisition layer or a combination of a wicking and distribution layer, and the second additional layer 286 is selected from a combination of a wicking and distribution layer or a storage layer. The first and second central fibrous layers 284 may be the same or different, and may include varying degrees and amounts of SAP.

The preferred embodiment illustrated in Figure 8 is essentially an inverted absorbent laminate core 28 of Figure 7. Here, the absorbent laminate core 28 preferably comprises an upper layer 280, and in descending order below the upper layer 280, a first central fibrous layer 284, a first additional layer, a second central fibrous layer 284, a second additional layer 286, and lower layer 282. Preferably, the first additional layer 286 is a combination of a wicking and distribution layer, and the second additional layer 286 is either a combination of a wicking and distribution layer or a storage layer. Again, the first and second central fibrous layers 284 may be the same or different, and may include varying degrees and amounts of SAP.

Figure 9 depicts a preferred embodiment of the invention that includes a fragmented layer. The absorbent laminate core 28 shown in Figure 9 includes an upper layer 280, a fragmented additional layer 286, a central fibrous layer 284, and a lower layer 282. Again, it is preferred that upper layer 280 and lower layer 282 are tissue layers. In the embodiment illustrated in Figure 9, it is preferred that the fragmented additional layer 286 be comprised of a fragmented wicking layer that is fragmented in the lateral direction 102. That is, additional layer 286 runs the entire length of absorbent laminate core 28 in the longitudinal direction (100), but is fragmented along the width, or vertical direction 102.

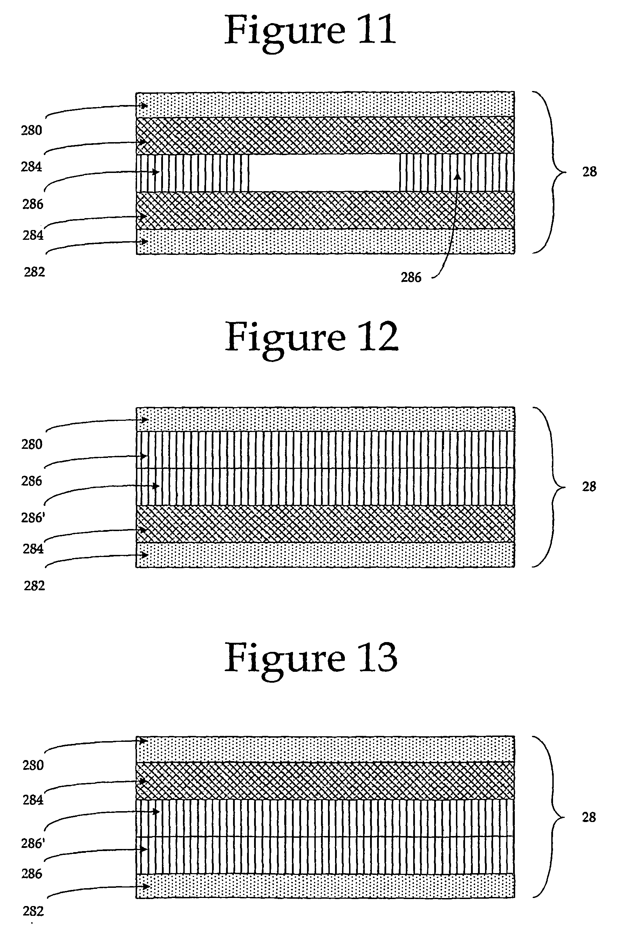

The preferred embodiment shown in Figure 10 is essentially the inverse of that shown in Figure 9 whereby the fragmented additional layer 286 is disposed between central fibrous layer 284 and lower layer 282. It is preferred that fragmented additional layer 286 be comprised of a fragmented wicking or storage layer. Figure 11 illustrates an embodiment whereby two central fibrous layers 284 are disposed between upper layer 280 and lower layer 282, and a fragmented additional layer 286 is disposed between the two central fibrous layers 284. In this embodiment, it is preferred that fragmented additional layer 286 be comprised of a fragmented wicking layer. Again, the first and second central fibrous layers 284 may be the same or different, and may include varying degrees and amounts of SAP.

Figures 12 and 13 illustrate embodiments of the invention that include two additional layers 286, 286'. In these embodiments, the two additional layers 286, 286' are different layers having different properties. Hence, if additional layer 286 is a fluid acquisition layer, then additional layer 286' is not a fluid acquisition layer but may be any of the other aforementioned layers. Figure 12 illustrates the preferred embodiment whereby two additional layers 286, 286' are disposed between upper layer 280 and central fibrous layer 284. It is preferred that additional layer 286 is a fluid acquisition layer, and additional layer 286' is a combination of a wicking and distribution layer, or it is a storage layer. Figure 13 illustrates the preferred embodiment whereby two additional layers 286, 286' are disposed between central fibrous layer 284 and lower layer 282. It is preferred that additional layer 286 is a combination of a wicking and distribution layer, and additional layer 286' is a storage layer.

It is contemplated in the present invention that the absorbent laminate core 28 be folded as it is disposed between the top sheet 24 and back sheet 26. Absorbent laminate core 28 can be folded in any suitable manner, including any and all of those disclosed in U.S. Patent No. 6,068,620. Figure 16 illustrates various preferred configurations for absorbent laminate core 28 to be folded in the invention, whereby in the figure, the respective layers of the absorbent laminate core 28 are not shown for purposes of clarity. Those skilled in the art will appreciate that the absorbent laminate core 28 can be folded such that the adjacent sides are touching one another, or so that channels are formed in certain areas. For example, the first folded embodiment in Figure 16 is the standard "C" fold. Here, the curled ends may be spaced apart to form a channel there between, and the lower edges of the curled ends may be disposed adjacent the upper edges of the bottom portion of the folded article. Alternatively, another absorbent material, or another absorbent laminate core 28 may be disposed in the space formed by the standard "C" fold illustrated in Figure 16.

The same considerations may be given to the "G" fold and the "U" fold found below the "C" fold of Figure 16. The spaces formed by these folds may be filled with another absorbent material, another absorbent laminate core 28, or the folds may be made tight enough so that little or no space is formed. Other possible arrangements include a "Z" fold, and a pleated absorbent laminate core 28, as shown at the bottom of Figure 16.

The absorbent laminate core 28 of the preferred embodiments is particularly ideal for narrow crotch diapers and training pants. Narrow crotch training pants either must typically sacrifice absorbent capacity at the narrowed portion as a result of reduced absorbent surface area, or must alternatively provide a thicker absorbent core to compensate for the reduced surface area. As the thickness of the core increases, comfort, fit and wearability decrease. By using the high absorbency absorbent laminate core 28 of the preferred embodiments in a narrow crotch absorbent garment, the absorbent capacity through the central crotch area is not sacrificed while comfort fit and wearability are improved.

Other embodiments, uses, and advantages of the invention will be apparent to those skilled in the art from consideration of the specification and practice of the invention disclosed herein. The specification should be considered exemplary only, and the scope of the invention is accordingly intended to be limited only by the following claims.

## Claims

1. An absorbent article having a longitudinal dimension (100) and a lateral dimension (102) comprising:
a top sheet (24);
a back sheet (26), whereby the top sheet (24) and the back sheet (26) form a first waist region (12), a second waist region (14) longitudinally opposite the first waist region (12), and a crotch region (16) there between;
an absorbent laminate core (28) at least partially disposed between the top sheet (24) and the back sheet (26);
the absorbent laminate core (28) comprising at least four layers whereby two of the layers are outer layers (280, 282) comprising an upper layer (280) and a lower layer (282), and one of the inner layers disposed between the upper layer and lower layer is a central fibrous layer (284) containing tow fibers and from 30 to 95% by weight super absorbent polymer (SAP),
whereby the absorbent laminate core (28) comprises at least one additional inner layer (286) disposed between the upper layer (280) and lower layer (282), the additional inner layer (286) being selected from the group consisting of a fluid acquisition layer, a distribution layer, a wicking layer, and combinations and fragments thereof.

2. The absorbent article of claim 1, further comprising at least one fastening element (34) attached to a lateral edge (18) of the first waist region (12); and one or more target devices (38) attached to the article in the second waist region, where at least one fastening element and the one or more target devices are capable of attaching to one another, the one or more target devices being located so that the first waist region and second waist region of the garment may be joined to one another to secure the garment on a wearer.

3. The absorbent article of claim 1 or 2, further comprising elastic leg gathers (30) comprising one or more elastic materials disposed adjacent the lateral edge of the crotch region, and standing leg gathers (40) disposed on the top sheet adjacent the lateral edge of the crotch region.

4. The absorbent article of claim 2 or 3, wherein the at least one fastening element comprises a hook portion of a hook and loop fastener and the one or more target devices comprise the loop portion of a hook and loop fastener, or wherein the at least one fastening element is an adhesive tape and the one or more target devices comprise a tape receiving surface.

5. The absorbent article of claim 2 or claim 3, wherein the at least one fastening element is comprised of a pair of laterally extending tabs (34) disposed on the lateral edges of the first waist region, whereby the laterally extending tabs each include at least one fastening element.

6. The absorbent article of any of claims 1 to 5, wherein the absorbent laminate core comprises one additional layer (286), and the layer is selected from a fluid acquisition layer, a distribution layer, a wicking layer, a fragmented layer, and a combination of a wicking layer and a distribution layer.

7. The absorbent article of claim 1, wherein the absorbent laminate core comprises two additional layers.

8. The absorbent article of any of the preceding claims, wherein the or one of the additional layer(s) is disposed between the central fibrous layer and the lower layer, the additional layer being selected from an optionally fragmented wicking layer, or the or one of the additional layer(s) is disposed between the central fibrous layer and the upper layer, the additional layer being selected from a fluid acquisition layer, a combination of a wicking and distribution layer, or a fragmented wicking layer.

9. The absorbent article of any of the preceding claims, wherein a second central fibrous layer is disposed between the first central fibrous layer and the lower layer; and
(a) the or one of the additional layer(s) is disposed between the first and second central fibrous layers, whereby the additional layer is selected from the group consisting of a combination of a wicking and distribution layer, a fragmented layer selected from a fragmented wicking layer, and/or
(b) a tissue layer is disposed between the first and second central fibrous layers.

10. The absorbent article of claim 1, wherein a first additional layer of said at least one additional layer is disposed between the central fibrous layer and the upper layer, the first additional layer being selected from a fluid acquisition layer or a combination of a wicking and distribution layer; and
a second additional layer of said at least one additional layer is disposed between the central fibrous layer and the lower layer, the second additional layer being selected from a combination of a wicking and distribution layer.

11. The absorbent article of claim 10, wherein a second central fibrous layer is disposed between the first central fibrous layer and the lower layer, and
the second additional layer is disposed between the first and second central fibrous layers.

12. The absorbent article of claim 1, wherein a second central fibrous layer is disposed between the first central fibrous layer and the lower layer;
a first additional layer of said at least one additional layer is disposed between the first and second central fibrous layers, the first additional layer being a combination of a wicking and distribution layer; and
a second additional layer of said at least one additional layer is disposed between the second central fibrous layer and the lower layer, the second additional layer being selected from a combination of a wicking and distribution layer.

13. The absorbent article of claim 1, wherein a first additional layer of said at least one additional layer is disposed between the upper and lower layers, the first additional layer being a fluid acquisition layer;
a second additional layer of said at least one additional layer is disposed between the first additional layer and the lower layer, the second additional layer being selected from a combination of a wicking and distribution layer; and
a central fibrous layer is disposed between the second additional layer and the lower layer.

14. The absorbent article of claim 1, wherein a first additional layer is disposed between the central fibrous layer and the lower layer, the first additional layer being a combination of a wicking and distribution layer; and
second additional layer is disposed between the first additional layer and the lower layer, the second additional layer being a storage layer.

15. The absorbent article of any of the preceding claims, wherein the central fibrous layer has a SAP efficiency of at least 80%.

16. The absorbent article of any of the preceding claims, wherein the central fibrous layer comprises fibers selected from the group consisting of cellulose acetate fibers, rayon fibers, LYOCELL fibers, polyacrylonitrile fibers, cotton fibers and cotton linter fibers.

17. The absorbent article of claim 16, wherein the central fibrous layer comprises cellulose acetate tow fibers.

18. The absorbent article of any of the preceding claims, wherein the central fibrous layer further comprises up to 10% by weight fluff wood pulp fibers and/or particulate additives.

19. The absorbent article of claim 18, wherein the central fibrous layer comprises particulate additives which comprise insoluble, hydrophilic polymers having particle diameters of 100 pm or less and/or which are selected from the group consisting of potato, corn, wheat, and rice starches, and partially cooked or modified starches.

20. A method of making an absorbent article comprising:
a) preparing a top sheet and a back sheet;
b) preparing an absorbent laminate core by:
b1) preparing an upper layer and a lower layer;
b2) preparing a central fibrous layer containing tow fibers and from 30 to 95% by weight of superabsorbent polymer particles (SAP), and at least partially disposing the central fibrous layer between the upper layer and lower layer; and
b3) preparing at least one additional layer selected from the group consisting of a fluid acquisition layer, a distribution layer, a wicking layer, and combinations and fragments thereof, and disposing the at least one additional layer between the upper and lower layers; and
c) disposing the absorbent laminate core between the top sheet and the back sheet,
whereby the top sheet, back sheet, and absorbent laminate core are prepared and arranged such that the top sheet and the back sheet form a first waist region, a second waist region longitudinally opposite the first waist region, and a crotch region between the waist regions.

21. The method of claim 20, further comprising
d) attaching at least one fastening element to lateral edges of the first waist region; and
e) preparing at least one target device and attaching the at least one target device to the article in the second waist region, where the at least one fastening element and the at least one target device are capable of attaching to one another, the at least one target device being located so that the first waist region and second waist region of the article may be joined to one another to secure the article on a wearer.

22. The method of claim 21, wherein the at least one fastening element comprises a hook portion of a hook and loop fastener and the at least one target device comprises the loop portion of a hook and loop fastener or wherein the at least one fastening element is an adhesive tape and the at least one target device comprises a tape receiving surface.

23. The method of claim 22, wherein the at least one fastening element is comprised of a pair of laterally extending tabs disposed on the lateral edges of the first waist region, whereby the laterally extending tabs each include at least one fastening element.

24. The method of any of claims 20 to 23, wherein the central fibrous layer comprises from about 50% to about 95% by weight super absorbent polymer (SAP).

25. The method of any of claims 20 to 24, wherein the central fibrous layer comprises fibers selected from the group consisting of cellulose acetate fibers, rayon fibers, LYOCELL fibers, polyacrylonitrile fibers, cotton fibers and cotton linter fibers.

26. The method of claim 25, wherein the central fibrous layer comprises cellulose acetate tow fibers.

27. The method of any of claims 20 to 26, wherein the central fibrous layer further comprises up to 10% by weight fluff wood pulp fibers and/or particulate additives.

28. The method of claim 27, wherein the central fibrous layer comprises particulate additives which comprise insoluble, hydrophilic polymers having particle diameters of 100 pm or less, and/or which are selected from the group consisting of potato, corn, wheat, and rice starches, and partially cooked or modified starches.

## Patentansprüche

1. Absorbierender Gegenstand mit einer Längsabmessung (100) und einer Querabmessung (102), umfassend:
eine Deckschicht (26);
eine rückseitige Schicht (26), wobei die Deckschicht (24) und die rückseitige Schicht (26) einen ersten Bundbereich (12), einen zweiten Bundbereich (14), der dem ersten Bundbereich (12) in Längsrichtung gegenüberliegt, und einen dazwischen liegenden Schrittbereich (16) bilden;
einen absorbierenden Laminatkern (28), der zumindest teilweise zwischen der Deckschicht (24) und der rückseitigen Schicht (26) angeordnet ist;
wobei der absorbierende Laminatkern (28) wenigstens vier Schichten
umfasst, wobei zwei der Schichten äußere Schichten (280,282) sind, die eine obere Schicht (280) und eine untere Schicht (282) umfassen, und eine der inneren Schichten, die zwischen der oberen Schicht und der unteren Schicht angeordnet ist, eine mittlere Faserschicht (284) ist, die Tow- oder Endlosfasern und 30 bis 95 Gew.-% superabsorbierendes Polymer (SAP) enthält;
wobei der saugfähige Laminatkern (28) zumindest eine zusätzliche innere Schicht (286) aufweist, die zwischen der oberen Schicht (280) und der unteren Schicht (282) angeordnet und aus der Gruppe ausgewählt ist, die aus einer Flüssigkeitsaufnahmeschicht, einer Verteilerschicht, einer Dochtschicht sowie Kombinationen und Fragmenten davon besteht.

2. Absorbierender Gegenstand nach Anspruch 1, ferner umfassend zumindest ein an einem seitlichen Rand (18) des ersten Bundbereiches (12) angebrachtes Befestigungselement (34); und eine oder mehrere in dem zweiten Bundbereich an dem Gegenstand befestigte Aufnahmevorrichtungen (38),
wobei wenigstens ein Befestigungselement und die eine oder mehreren Aufnahmevorrichtung(en) aneinander befestigt werden können, und wobei die eine oder mehreren Aufnahmevorrichtung(en) so positioniert sind, dass der erste Bundbereich und der zweite Bundbereich des Kleidungsstücks miteinander verbunden werden können, um das Kleidungsstück an einem Träger sicher zu halten.

3. Absorbierender Gegenstand nach Anspruch 1 oder 2, ferner umfassend elastische Beinabschlüsse oder -falten (30), welche ein oder mehrere elastische Materialien umfassen, die sich benachbart zum seitlichen Rand des Schrittbereichs befinden, und stehende Beinabschlüsse oder-falten (40), die unmittelbar benachbart zum Seitenrand des Schrittbereiches auf der Deckschicht angebracht sind.

4. Absorbierender Gegenstand nach Anspruch 2 oder 3, worin das zumindest eine Befestigungselement einen Haken-Abschnitt eines Haken- und Ösenverschlusses umfasst und die eine oder mehrere Aufnahmevorrichtung(en) den Ösen-Abschnitt eines Haken- und Ösenverschlusses umfasst, oder worin das zumindest eine Befestigungselement ein Klebeband ist und die eine oder mehreren Aufnahmevorrichtung(en) eine Klebeband-Aufnahmefläche umfasst/umfassen.

5. Absorbierender Gegenstand nach Anspruch 2 oder Anspruch 3, worin das zumindest eine Befestigungselement aus einem Paar sich seitlich erstreckender Laschen (34) besteht, die auf den seitlichen Rändern des ersten Bundbereiches angebracht sind, wobei jede der sich seitlich erstreckenden Laschen zumindest ein Befestigungselement umfasst.

6. Absorbierender Gegenstand nach einem der Ansprüche 1 bis 5, worin der absorbierende Laminatkern eine zusätzliche Schicht (286) umfasst und die Schicht aus einer Flüssigkeitsaufnahmeschicht, einer Verteilerschicht, einer Dochtschicht, einer fragmentierten Schicht und einer Kombination aus Dochtschicht und Verteilerschicht ausgewählt ist.

7. Absorbierender Gegenstand nach Anspruch 1, worin der absorbierende Laminatkern zwei zusätzliche Schichten umfasst.

8. Absorbierender Gegenstand gemäß einem der voranstehenden Ansprüche, worin die oder eine der zusätzliche(n) Schicht(en) zwischen der mittleren Faserschicht und der unteren Schicht angebracht und die zusätzliche Schicht aus einer optional fragmentierten Dochtschicht ausgewählt ist, oder worin die oder eine der zusätzliche(n) Schicht(en) zwischen der mittleren Faserschicht und der oberen Schicht angebracht und die zusätzliche Schicht aus einer Flüssigkeitsaufnahmeschicht, einer Kombination aus Dochtschicht und Verteilerschicht oder einer fragmentierten Dochtschicht ausgewählt ist.

9. Absorbierender Gegenstand nach einem der voranstehenden Ansprüche, worin eine zweite mittlere Faserschicht zwischen der ersten mittleren Faserschicht und der unteren Schicht angebracht ist und
(a) die oder eine der zusätzliche(n) Schicht(en) zwischen der ersten und zweiten mittleren Faserschicht angebracht ist, wobei die zusätzliche Schicht aus der Gruppe ausgewählt ist, die aus einer Kombination aus Docht- und Verteilerschicht und einer aus einer fragmentierten Dochtschicht ausgewählten fragmentierten Schicht besteht, und/oder
(b) eine Textilschicht zwischen der ersten und der zweiten mittleren Faserschicht angebracht ist.

10. Absorbierender Gegenstand nach Anspruch 1, worin eine erste zusätzliche Schicht der genannten, zumindest einen zusätzlichen Schicht zwischen der mittleren Faserschicht und der oberen Schicht angebracht ist, wobei die erste zusätzliche Schicht aus einer Flüssigkeitsaufnahmeschicht oder einer Kombination aus Docht- und Verteilerschicht ausgewählt ist; und eine zweite zusätzliche Schicht der genannten, zumindest einen zusätzlichen Schicht zwischen der mittleren Faserschicht und der unteren Schicht angebracht ist, wobei die zweite zusätzliche Schicht aus einer Kombination aus Docht- und Verteilerschicht ausgewählt wird.

11. Absorbierender Gegenstand nach Anspruch 10, worin eine zweite mittlere Faserschicht zwischen der ersten mittleren Faserschicht und der unteren Schicht angebracht ist, und die zweite zusätzliche Schicht zwischen der ersten und der zweiten mittleren Faserschicht angebracht ist.

12. Absorbierender Gegenstand nach Anspruch 1, worin eine zweite mittlere Faserschicht zwischen der ersten mittleren Faserschicht und der unteren Schicht angebracht ist;
eine erste zusätzliche Schicht der genannten, zumindest einen zusätzlichen Schicht zwischen der ersten und der zweiten mittleren Faserschicht angebracht ist, wobei die erste zusätzliche Schicht eine Kombination aus einer Docht- und Verteilerschicht ist; und
eine zweite zusätzliche Schicht der genannten, zumindest einen zusätzlichen Schicht zwischen der zweiten mittleren Faserschicht und der unteren Schicht angebracht ist, wobei die zweite zusätzliche Schicht aus einer Kombination von Docht- und Verteilerschicht ausgewählt ist.

13. Absorbierender Gegenstand nach Anspruch 1, worin eine erste zusätzliche Schicht der genannten, wenigstens einen zusätzlichen Schicht zwischen der oberen und der unteren Schicht angebracht ist, wobei die erste zusätzliche Schicht eine Flüssigkeitsaufnahmeschicht ist;
eine zweite zusätzliche Schicht der genannten, zumindest einen zusätzlichen Schicht zwischen der ersten zusätzlichen Schicht und der unteren Schicht angebracht ist, wobei die zweite zusätzliche Schicht aus einer Kombination von Docht- und Verteilerschicht ausgewählt ist; und
eine mittlere Faserschicht zwischen der zweiten zusätzlichen Schicht und der unteren Schicht angebracht ist.

14. Absorbierender Gegenstand nach Anspruch 1, worin eine erste zusätzliche Schicht zwischen der mittleren Faserschicht und der unteren Schicht angebracht ist, wobei die erste zusätzliche Schicht eine Kombination aus einer Docht- und Verteilerschicht ist; und
eine zweite zusätzliche Schicht zwischen der ersten zusätzlichen Schicht und der unteren Schicht angebracht ist, wobei die zweite zusätzliche Schicht eine Speicherschicht ist.

15. Absorbierender Gegenstand nach einem der voranstehenden Ansprüche, worin die mittlere Faserschicht eine SAP-Wirksamkeit von wenigstens 80% besitzt.

16. Absorbierender Gegenstand nach einem der voranstehenden Ansprüche, worin die mittlere Faserschicht Fasern umfasst, welche aus der Gruppe ausgewählt sind, die aus Celluloseacetatfasern, Reyonfasern (Viskosefasern), LYOCELL-Fasern, Polyacrylnitrilfasern, Baumwollfasern und Baumwolllinters-Fasern besteht.

17. Absorbierender Gegenstand nach Anspruch 16, worin die mittlere Faserschicht Tow- oder Endlosfasern aus Celluloseacetat umfasst.

18. Absorbierender Gegenstand nach einem der voranstehenden Ansprüche, worin die mittlere Faserschicht ferner bis zu 10 Gew.-% Fasern aus Zellstoffflocken und/oder teilchenförmige Zusätze umfasst.

19. Absorbierender Gegenstand nach Anspruch 18, worin die mittlere Faserschicht teilchenförmige Zusätze aufweist, die unlösliche, hydrophile Polymere mit Teilchendurchmessern von 100 µm oder weniger umfassen, und/oder die aus der Gruppe ausgewählt sind, die aus Kartoffel-, Mais-, Weizen- und Reisstärken und teilweise aufgeschlossenen oder modifizierten Stärken besteht.

20. Verfahren zur Herstellung eines absorbierenden Gegenstandes, umfassend:
a) Herstellen einer Deckschicht und einer rückseitigen Schicht;
b) Herstellen eines absorbierenden Laminatkerns durch:
b1) Herstellen einer oberen Schicht und einer unteren Schicht;
b2) Herstellen einer mittleren Faserschicht, die Tow- oder Endlosfasern und 30 bis 95 % Gew.-% Teilchen aus superabsorbierendem Polymer (SAP) enthält, und wenigstens teilweise erfolgendes Anordnen der mittleren Faserschicht zwischen der oberen und der unteren Schicht; und
b3) Herstellen zumindest einer zusätzlichen Schicht, ausgewählt aus der Gruppe, die aus einer Flüssigkeitsaufnahmeschicht, einer Verteilerschicht, einer Dochtschicht und Kombinationen und Fragmenten davon besteht, und Anordnen der zumindest einen zusätzlichen Schicht zwischen der oberen und der unteren Schicht; und
c) Anbringen des absorbierenden Laminatkerns zwischen der Deckschicht und der rückseitigen Schicht,
wobei die Deckschicht, die rückseitige Schicht und der absorbierende Laminatkern derart hergestellt und angeordnet werden, dass die Deckschicht und die rückseitige Schicht einen ersten Bundbereich, einen dem ersten Bundbereich in Längsrichtung gegenüberliegenden zweiten Bundbereich und einen Schrittbereich zwischen den Bundbereichen bilden.

21. Verfahren nach Anspruch 20, ferner umfassend:
d) Anbringen zumindest eines Befestigungselements an seitlichen Rändern des ersten Bundbereichs; und
(e) Herstellen zumindest einer ersten Aufnahmevorrichtung und Befestigen der zumindest einen Aufnahmevorrichtung an dem Gegenstand in dem zweiten Bundbereich, wobei das wenigstens eine Befestigungselement und die zumindest eine Aufnahmevorrichtung aneinander befestigt werden können, wobei die zumindest eine Aufnahmevorrichtung so positioniert ist, dass der erste Bundbereich und der zweite Bundbereich des Gegenstandes miteinander verbunden werden können, um den Gegenstand an einem Träger sicher zu halten.

22. Verfahren nach Anspruch 21, worin das zumindest eine Befestigungselement einen Hakenabschnitt eines Haken- und Ösenverschlusses umfasst und die zumindest eine Aufnahmevorrichtung den Ösenabschnitt eines Haken- und Ösenverschlusses umfasst, oder worin das zumindest eine Befestigungselement ein Klebeband ist und die zumindest eine Aufnahmevorrichtung eine Klebeband-Aufnahmefläche umfasst.

23. Verfahren nach Anspruch 22, worin das zumindest eine Befestigungselement aus einem Paar sich seitlich erstreckender Laschen besteht, die an den seitlichen Rändern des ersten Bundbereichs angebracht sind, wobei jede der sich seitlich erstreckenden Laschen jeweils mindestens ein Befestigungselement aufweist.

24. Verfahren nach einem der Ansprüche 20 bis 23, worin die mittlere Faserschicht etwa 50 bis etwa 95 Gew.-% superabsorbierendes Polymer (SAP) aufweist.

25. Verfahren nach einem der Ansprüche 20 bis 24, worin die mittlere Faserschicht Fasern aufweist, die aus der Gruppe ausgewählt sind, welche aus Celluloseacetatfasern, Reyonfasern (Viskosefasern), LYOCELL-Fasern, Polyacrylnitrilfasern, Baumwollfasern und Baumwolllinters-Fasern besteht.

26. Verfahren nach Anspruch 25, worin die mittlere Faserschicht Tow- oder Endlosfasern aus Celluloseacetat umfasst.

27. Verfahren nach einem der Ansprüche 20 bis 26, worin die mittlere Faserschicht ferner bis zu 10 Gew.-% Fasern aus Zellstoffflocken und/oder teilchenförmige Zusätze umfasst.

28. Verfahren nach Anspruch 27, worin die mittlere Faserschicht teilchenförmige Zusätze aufweist, die unlösliche, hydrophile Polymere mit Teilchendurchmessern von 100 µm oder weniger umfassen, und/oder die aus der Gruppe ausgewählt sind, welche aus Kartoffel-, Mais-, Weizen- und Reisstärken und teilweise aufgeschlossenen oder modifizierten Stärken besteht.

## Revendications

1. Un article absorbant ayant une dimension longitudinale (100) et une dimension transversale (102) comprenant :
une feuille supérieure (24) ;
une feuille de base (26), dans lequel la feuille supérieure (24) et la feuille de base (26) forment une première région de taille (12), une seconde région de taille (14) longitudinalement opposée à la première région de taille (12), et une région d'entrejambes interposée ;
un noyau absorbant laminé (28) disposé au moins partiellement entre la feuille supérieure (24) et la feuille de base (26) ;
le noyau absorbant laminé (28) comprenant au moins quatre couches dans lesquelles deux des couches sont des couches externes (280, 282) comprenant une couche supérieure (280) et une couche inférieure (282), et une des couches internes disposées entre la couche supérieure et la couche inférieure est une couche centrale fibreuse (284) contenant des fibres d'étoupe et de 30 à 95% en poids de polymère super absorbant (SAP),
dans lequel le noyau absorbant laminé (28) comprend au moins une couche interne additionnelle (286) disposée entre la couche supérieure (280) et la couche inférieure (282), la couche interne additionnelle (286) étant sélectionnée dans le groupe composé d'une couche d'acquisition de fluide, une couche de distribution, une couche mèche et leurs combinaisons et leurs fragments.

2. L'article absorbant selon la revendication 1, comprenant en outre au moins un élément de fermeture (34) attaché à un bord latéral (18) de la première région de taille (12); et un ou plusieurs dispositifs cibles attachés à l'article dans la seconde région de taille, dans lequel au moins un élément de fermeture et le ou les dispositifs cibles sont capables de s'attacher l'un à l'autre, le ou les dispositifs cibles étant situé de manière à ce que la première région de taille et la seconde région de taille du vêtement peuvent être joints l'un à l'autre pour immobiliser le vêtement sur un usager.

3. L'article absorbant selon la revendication 1 ou 2, comprenant en outre des fronces de jambe élastiques (30) comprenant un ou plusieurs matériaux élastique disposés de façon adjacente au bord latéral de la région d'entrejambe, et coopérant avec les fronces de jambe (40) disposés sur la feuille supérieure adjacente au bord latéral de la région d'entrejambe.

4. L'article absorbant de la revendication 2 ou 3, dans lequel l'élément de fermeture comprend une portion crochet d'une fermeture à boucle et crochet et le ou les dispositifs cibles comprennent une portion boucle d'une fermeture à boucle et crochet, ou dans lequel l'élément de fermeture est une bande adhésive et le ou les dispositifs cibles comprennent une surface de réception de la bande adhésive.

5. L'article absorbant selon la revendication 2 ou 3, dans lequel l'élément de fermeture comprend une paire de pattes s'étendant latéralement, disposées sur les bords latéraux de la première région de taille, et comprenant chacune au moins un élément de fermeture.

6. L'article absorbant selon l'une quelconque des revendications 1 à 5,
dans lequel le noyau absorbant laminé comprend une couche additionnelle (286), et la couche est sélectionnée parmi une couche d'acquisition de fluide, une couche de distribution, une couche mèche, une couche fragmentée, et une combinaison d'une couche mèche et d'une couche de distribution.

7. L'article absorbant selon la revendication 1, dans lequel le noyau absorbant laminé comprend deux couches additionnelles.

8. L'article absorbant selon l'une quelconque des revendications précédentes, dans lequel la ou l'une des couches additionnelles est disposée entre la couche fibreuse centrale et la couche inférieure, la couche additionnelle étant sélectionnée parmi une couche mèche fragmentée optionnellement, ou la ou l'une des couches additionnelle est disposée entre la couche fibreuse centrale, la couche additionnelle étant sélectionnée parmi une couche d'acquisition de fluide, une combinaison d'une couche mèche et d'une couche de distribution, ou d'une couche mèche fragmentée.

9. L'article absorbant selon l'une quelconque des revendications précédentes, dans lequel une seconde couche fibreuse centrale est disposée entre la première couche fibreuse centrale et la couche inférieure ; et
(a) la ou l'une des couches additionnelles est disposée entre les première et seconde couches fibreuses centrales, dans lequel la couche additionnelle étant sélectionnée parmi le groupe composé d'une combinaison d'une couche mèche et d'une couche de distribution, une couche fragmentée sélectionnée à partir d'une couche mèche fragmentée, et/ou
(b) une couche de tissu est disposée entre les première et seconde couches fibreuses centrales.

10. L'article absorbant selon la revendication 1, dans lequel une première couche additionnelle de ladite au moins une couche additionnelle est disposée entre la couche fibreuse centrale et la couche supérieure, la première couche additionnelle étant sélectionnée parmi une couche d'acquisition de fluide ou une combinaison d'une couche mèche et d'une couche de distribution ; et une seconde couche additionnelle de ladite au moins une couche additionnelle est disposée entre la couche fibreuse centrale et la couche inférieure, la seconde couche additionnelle étant sélectionnée parmi une combinaison d'une couche mèche et d'une couche de distribution.

11. L'article absorbant selon la revendication 10, dans lequel la seconde couche fibreuse centrale est disposée entre la première couche fibreuse centrale et la couche inférieure, et la seconde couche additionnelle est disposée entre les première et seconde couches fibreuses centrales.

12. L'article absorbant selon la revendication 1, dans lequel une seconde couche fibreuse centrale est disposée entre la première couche fibreuse centrale et la couche inférieure ;
une première couche additionnelle de ladite au moins une couche additionnelle est disposée entre les première et seconde couches fibreuses centrales, la première couche additionnelle étant une combinaison d'une couche mèche et d'une couche de distribution ; et
une seconde couche additionnelle de ladite au moins couche additionnelle est disposée entre la seconde couche fibreuse centrale et la couche inférieure, la seconde couche additionnelle étant sélectionnée parmi une couche mèche et une couche de distribution.

13. L'article absorbant selon la revendication 1, dans lequel une première couche additionnelle de ladite au moins couche additionnelle est disposée entre les couches supérieure et inférieure, la première couche additionnelle étant une couche d'acquisition de fluide ;
une seconde couche additionnelle de ladite au moins une couche additionnelle est disposée entre la première couche additionnelle et la couche inférieure, la seconde couche additionnelle étant sélectionnée parmi une combinaison d'une couche mèche et d'une couche de distribution ; et une couche fibreuse centrale est disposée entre la seconde couche additionnelle et la couche inférieure.

14. L'article absorbant selon la revendication 1, dans lequel une première couche additionnelle est disposée entre la couche fibreuse centrale et la couche inférieure, la première couche additionnelle étant une combinaison d'une couche mèche et d'une couche de distribution ; et une seconde couche additionnelle est disposée entre la première couche additionnelle et la couche inférieure, la seconde couche additionnelle étant une couche de stockage.

15. L'article absorbant selon l'une quelconque des revendications précédentes, dans lequel la couche fibreuse centrale a une efficacité de SAP d'au moins 80%.

16. L'article absorbant selon l'une quelconque des revendications précédentes, dans lequel la couche fibreuse centrale comprend des fibres sélectionnées parmi le groupe composé de fibres d'acétate de cellulose, des fibres rayonne, des fibres LYOCELL, des fibres poly acrylonitrile, des fibres coton et des fibres linter de coton.

17. L'article absorbant selon la revendication 16, dans lequel la couche fibreuse centrale comprend des fibres d'étoupe d'acétate de cellulose.

18. L'article absorbant selon l'une quelconque des revendications précédentes, dans lequel la couche fibreuse centrale comprend en outre jusqu'à 10% en poids des flocons de fibres de pulpe de bois et/ou d'additifs particulaires.

19. L'article absorbant selon la revendication 18, dans lequel la couche fibreuse centrale comprend des additifs particulaires qui comprennent des polymères insolubles, hydrophiles ayant des diamètres de particule de 100µm ou moins et/ou qui sont sélectionnés parmi le groupe composé de pomme de terre, maïs, blé, et amidons de riz, et amidons partiellement préparés ou modifiés.

20. Méthode de fabrication d'un article absorbant comprenant :
a) la préparation d'une feuille supérieure et d'une feuille de base
b) la préparation d'un noyau absorbant laminé par :
b1) la préparation d'une couche supérieure et d'une couche inférieure
b2) la préparation d'une couche fibreuse centrale contenant des fibres d'étoupe et entre 30 à 95% en poids de particules de polymère super absorbant (SAP), et le positionnement au moins partiellement de la couche fibreuse centrale entre la couche supérieure et la couche inférieure ; et
b3) la préparation d'au moins une couche additionnelle sélectionnée dans le groupe composé d'une couche d'acquisition de fluide, une couche de distribution, une couche mèche, et leurs combinaisons et fragments, et le positionnement d'au moins la couche additionnelle entre les couche supérieure et inférieure ; et
c) le positionnement du noyau absorbant laminé entre la feuille supérieure et la feuille de base,
dans lequel le feuille supérieure, la feuille de base, et le noyau absorbant laminé sont préparés et arrangés de manière à ce que la feuille supérieure et la feuille de base forment une première région de taille, une seconde région de taille longitudinalement opposée à la première région de taille, et une région d'entrejambe entre les régions de taille.

21. La méthode selon la revendication 20, comprenant en outre
d) La fixation d'au moins un élément de fermeture aux bords latéraux de la première région de taille ; et
e) la préparation d'au moins un dispositif cible et la fixation d'au moins le dispositif cible à l'article dans la seconde région de taille, où l'élément de fermeture et au moins le dispositif cible sont capables de s'attacher l'un à l'autre, le dispositif cible étant situé de manière à ce que la première région de taille et la seconde région de taille peuvent être jointes l'une à l'autre pour immobiliser l'article sur l'usager.

22. La méthode selon la revendication 21, dans laquelle l'élément de fermeture comprend une portion crochet d'une attache à boucle et crochet et le dispositif cible comprend la portion boucle de l'attache à boucle et crochet ou dans lequel l'élément de fermeture est une bande adhésive et le dispositif cible comprend une surface de réception de la bande.

23. La méthode selon la revendication 22, dans laquelle l'élément d'attache est composé d'une paire de pattes s'étendant latéralement disposées sur le bord latéral de la première région de taille, et comprenant chacune au moins un élément de fermeture.

24. La méthode selon l'une des revendications 20 à 23, dans laquelle la couche fibreuse centrale comprend entre environ 50% et environ 95% en poids d'un polymère super absorbant (SAP).

25. La méthode selon l'une des revendications 20 à 24, dans laquelle la couche fibreuse centrale comprend des fibres sélectionnées dans le groupe composé de fibres d'acétate de cellulose, des fibres rayonne, des fibres LYOCELL, des fibres poly acrylonitrile, des fibres coton et des fibres linter de coton.

26. La méthode selon la revendication 25, dans laquelle la couche fibreuse centrale comprend des fibres d'étoupe d'acétate de cellulose.

27. La méthode selon l'une des revendications 20 à 26, dans laquelle la couche fibreuse centrale comprend en outre jusqu'à 10% en poids de flocons de fibres de pulpe de bois et/ou des additifs particulaires.

28. La méthode selon la revendication 27, dans laquelle la couche fibreuse centrale comprend des additifs particulaires qui comprennent des polymères insolubles, hydrophiles ayant des particules de diamètres de 100µm ou moins, et/ou qui sont sélectionnés dans le groupe composé de pomme de terre, maïs, blé, amidon de riz, et amidon partiellement cuisinés ou modifiés.
